(19) Europäisches Patentamt
European Patent Office
Office européen des brevets

(11) Numéro de publication: **0 074 307**
**A1**

(12) **DEMANDE DE BREVET EUROPEEN**

(21) Numéro de dépôt: 82401577.0

(22) Date de dépôt: 25.08.82

(51) Int. Cl.³: C 07 C 103/84
C 07 C 103/82, A 61 K 49/04
//C07C103/78, C07C103/76,
C07C103/46, C07C121/52,
C07D209/48

(30) Priorité: 28.08.81 FR 8116476

(43) Date de publication de la demande:
16.03.83 Bulletin 83/11

(84) Etats contractants désignés:
AT BE CH DE FR GB IT LI LU NL SE

(71) Demandeur: Société dite: GUERBET S.A.
16-24 Rue Jean-Chaptal
F-93609 Aulnay-sous-Bois(FR)

(72) Inventeur: Dimo, Ioana
23 Rue Du Commandant Mouchotte
F-94160 Saint Mande(FR)

(72) Inventeur: Bonnemain, Bruno
9 Rue de Reims
F-77290 Mitry Mory(FR)

(72) Inventeur: Hardouin, Michel Jean-Charles
88 Avenue Foch
F-94120 Fontenay-Sous-Bois(FR)

(72) Inventeur: Lautrou, Jean
21 Avenue Gambetta
F-94160 Saint Mande(FR)

(74) Mandataire: Bressand, Georges et al,
c/o CABINET LAVOIX 2 Place d'Estienne d'Orves
F-75441 Paris Cedex 09(FR)

(54) **Composés mixtes bromo-iodo benzéniques et produits de contraste les contenant.**

(57) Composés à structure iodobenzénique, utilisables comme produits opacifiants pour la radiographie, présentant une tolérance plus grande que celle des composés analogues polyiodés et une opacité du même ordre de grandeur que ces composés polyiodés, caractérisés en ce qu'une partie des atomes d'iode nucléaires présents dans les composés analogues polyiodés est remplacée par des atomes de brome.

EP 0 074 307 A1

Croydon Printing Company Ltd.

# Composés mixtes bromo-iodo benzéniques et produits de contraste les contenant.

La présente invention concerne des produits opacifiants utilisés pour la radiographie.

On utilise depuis longtemps comme produits opacifiants des composés iodobenzéniques présentant sur le noyau benzénique plusieurs atomes d'iode, en général 3 atomes d'iode par noyau benzénique, et divers autres substituants. Ces autres substituants sont des groupes pharmacologiquement acceptables qui permettent l'administration des composés chez l'homme et les animaux. Ces substituants sont généralement choisis pour conférer aux composés une hydrosolubilité suffisante en vue de l'administration de ces composés en solution aqueuse.

De multiples solutions ont été proposées jusqu'à présent pour augmenter la tolérance des composés iodobenzéniques utilisés comme produits opacifiants.

Un premier type de solution a consisté à synthétiser des structures présentant deux ou trois noyaux benzéniques triiodés (voir par exemple les brevets US-A-3 290 366 et GB 1 346 795).

Un second type de solution a consisté à choisir les substituants autres que les atomes d'iode de façon à obtenir une meilleure tolérance. En particulier, on s'est orienté vers des structures non ioniques, c'est-à-dire ne présentant pas de substituants ionisants tels que des groupes carboxy (voir par exemple les brevets DE-A-2 031 724 et FR-A-2 253 509).

Un troisième type de solution a consisté à synthétiser des composés di- ou tribenzéniques polyiodés dissymétriques présentant un seul groupe ionisant (voir par exemple le brevet US-A-4 014 986).

La Demanderesse a cherché à résoudre le problème de l'augmentation de la tolérance des composés iodobenzéniques d'une manière fondamentalement différente de ce qui a été fait jusqu'à présent.

Elle a découvert que, contrairement à ce que l'on pouvait attendre, si l'on remplace dans les composés iodobenzéniques connus une partie des atomes d'iode nucléaires par des atomes de brome, non seulement on augmente la tolérance des composés, mais on obtient une opacité du même ordre de grandeur.

Ce dernier fait est particulièrement surprenant car, normalement, on devait s'attendre à ce que la substitution d'atomes d'iode par des atomes de brome entraîne une diminution notable de l'opacité. En effet, il est connu que l'opacité d'un atome aux rayons X est sensiblement proportionnelle à la puissance 3 de son numéro atomique (J. Duheix, V. Bismuth, M. Laval-Jeantet - Traité de radiodiagnostic, vol. 1 - L'image radiologique, Masson et Cie, 1969). Le numéro atomique du brome est 35, alors que celui de l'iode est 53. L'opacité conférée par le brome devrait donc être trois à quatre fois plus faible. On pouvait donc s'attendre à ce que le remplacement d'une partie des atomes d'iode par des atomes de brome conduise à une réduction importante de l'opacité, à un point tel que le composé ne soit plus, en pratique, utilisable comme produit opacifiant. Au contraire, on peut obtenir par le remplacement partiel des atomes d'iode par des atomes de brome des opacités qui sont du même ordre de grandeur que celles obtenues avec les composés polyiodés correspondants. Il s'ajoute à ceci un avantage économique important car le brome est actuellement nettement moins onéreux que l'iode.

En conséquence, la présente invention a pour objet des composés à structure iodobenzénique, utilisables comme produits opacifiants pour la radiographie, présentant une tolérance plus grande que celle des composés polyiodés utilisables comme produits opacifiants en radiographie et une opacité du même ordre de grandeur que ces composés polyiodés, caractérisés en ce qu'une partie des atomes d'iode nucléaires présent dans les composés polyiodés est remplacée par des atomes de brome.

Dans ces composés, le nombre d'atomes de brome nucléaires représente avantageusement de 1/2 à 2/1 du nombre des atomes d'iode nucléaire. De préférence, le nombre d'atomes de brome est égal au nombre d'atomes d'iode dans ces composés. Il peut s'agir dans ce cas notamment de composés présentant deux noyaux benzéniques dont l'un est triiodé et dont l'autre est tribromé.

Les composés selon la présente invention peuvent notamment être les composés suivants :

I - Les composés de formule

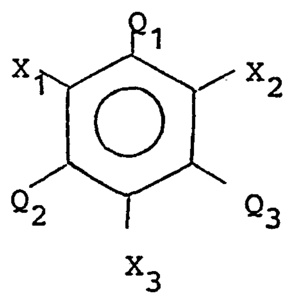

(I)

dans laquelle $X_1$, $X_2$ et $X_3$ sont choisis parmi I et Br, I et Br étant présents simultanément, et

$Q_1$, $Q_2$ et $Q_3$ sont des groupes pharmacologiquement acceptables.

II - Les composés de formule.

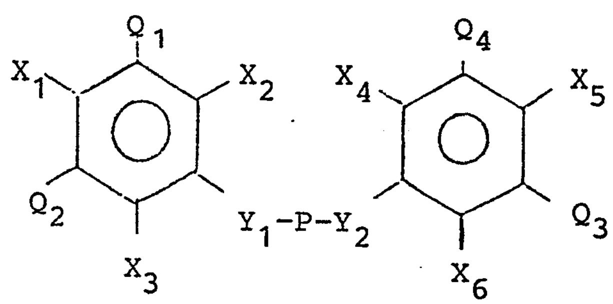

(II)

dans laquelle

$X_1$, $X_2$, $X_3$, $X_4$, $X_5$ et $X_6$ sont choisis parmi I et Br , I et Br étant présents simultanément, et

$Q_1$, $Q_2$, $Q_3$, $Q_4$, $Y_1$, $Y_2$ et P sont des groupes pharmacologiquement acceptables.

Parmi les composés de formule II une classe avantageuse de composés est celle de formule

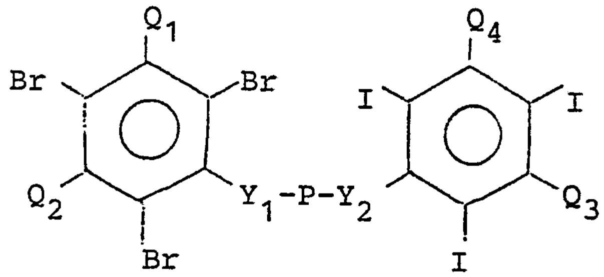

(IIa)

dans laquelle

$Q_1$, $Q_2$, $Q_3$, $Q_4$, $Y_1$, $Y_2$ et P sont des groupes pharmacologiquement acceptables.

III - Les composés de formule

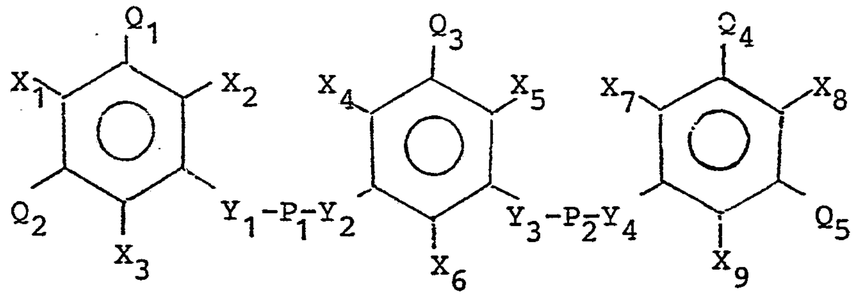

(III)

dans laquelle

$X_1$, $X_2$, $X_3$, $X_4$, $X_5$, $X_6$, $X_7$, $X_8$ sont choisis parmi I et Br, I et Br étant présents simultanément, et $Q_1$, $Q_2$, $Q_3$, $Q_4$, $Q_5$, $Y_1$, $Y_2$, $Y_3$, $Y_4$, $P_1$ et $P_2$ sont des groupes pharmacologiquement acceptables.

IV - Les composés de formule

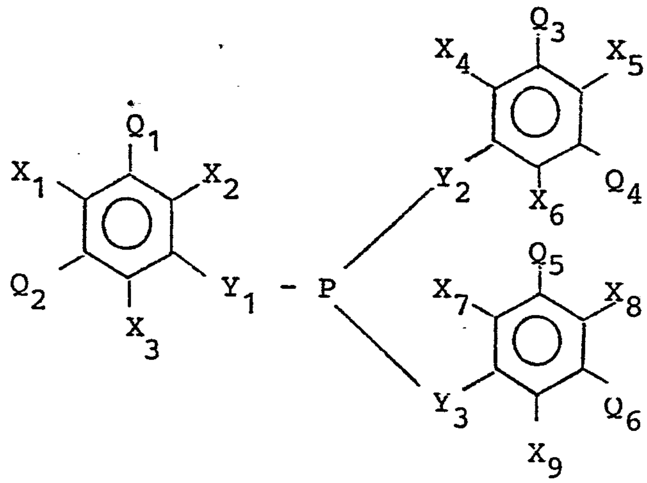

(IV)

dans laquelle

$X_1$, $X_2$, $X_3$, $X_4$, $X_5$, $X_6$, $X_7$, $X_8$, $X_9$ sont choisis parmi I et Br, I et Br étant présents simultanément, et

 

$Q_1$, $Q_2$, $Q_3$, $Q_4$, $Q_5$, $Q_6$, $Y_1$, $Y_2$, $Y_3$ et P étant des groupes pharmaceutiquement acceptables.

On donnera ci-après des exemples de groupes de type Q, Y, Z et P.

1) Groupes de type Q.

a) l'hydrogène

b) des groupes aminés hydrophiles tels que

⊖ des groupes A de formule

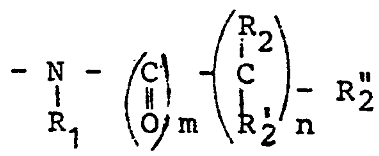

dans laquelle

m = 0, 1 ou 2

n = 0 à 6

et les radicaux $R_1$, $R_2$, $R'_2$ et $R''_2$ sont des groupes de formule $-(CHZ)_aT$ avec a = 0 à 5

Z = H ou OH

T = H, OH ou COOH

$-\left[(CH_2)_b-O(CH_2)_d-\right]_p H$ avec b = 1 à 5

d = 1 à 5

p = 1 à 5

- reste de sucre

Comme exemples typiques de tels groupes, on peut citer les groupes de formule :

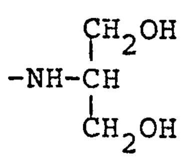 ; 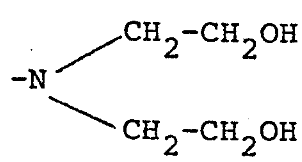 ; 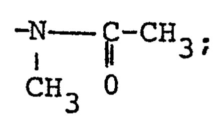 ;

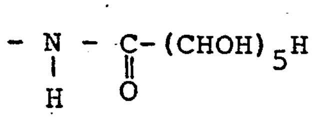 ; 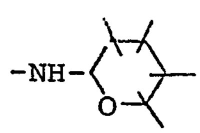

⊖ des groupes de formule

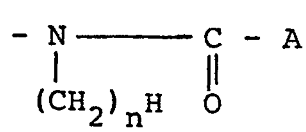

dans laquelle $n = 0$ à 3
et A est un groupe azoté tel que défini ci-dessus.

⊖ des groupes de formule

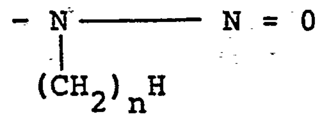

avec $n = 0$ à 3

⊖ des groupes de formule

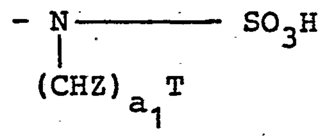

dans laquelle $Z = H$ ou OH
$T = H$ ou OH
$a_1 = 0$ à 3

c) des groupes éther ou ester tels que

⊖ des groupes alcoxy en $C_1$ à $C_6$ éventuellement substitués, notamment par des groupes carbonés ou des groupes aminés tels que le groupe A défini précédemment;

⊖ des groupes de formule

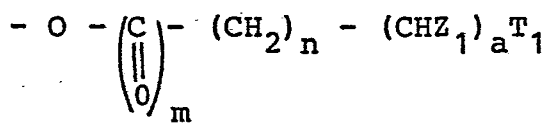

dans laquelle $m = 0$, 1 ou 2
$n = 0$ à 6
$a = 0$ à 6
$Z_1 = H$, OH ou $NH_2$
$T_1 = H$, OH ou $NH_2$

⊖ des groupes de formule 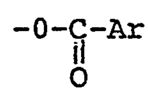

dans laquelle Ar représente un radical aromatique

⊖ des groupes de formule

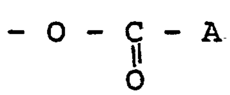 ou 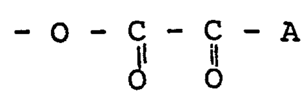

dans lesquelles A est tel que défini précédemment.

d) des groupes sulfonylés tels que

⊖ le groupe $-SO_3H$

⊖ les groupes de formule $-SO_2-A$ dans laquelle A est tel que défini précédemment.

e) des groupes liés par un carbone tels que

⊖ des groupes de formule $-(CHZ)_aT$

dans laquelle $a = 0$ à 6

$Z = H$ ou $OH$

$T = H$ ou $OH$

⊖ des groupes de formule $-(CH_2)_n-O-Ar$ dans laquelle Ar représente un radical aromatique

⊖ des groupes de formules

$-(CH_2)_n-C(=O)-H$ ou $-(CH_2)_n-CH(OH)_2$

$-(CH_2)_n-C(=O)-OH$

$-(CH_2)_n-C(=O)-A$

dans lesquelles $n = 0$ à 6

A est tel que défini précédemment

⊖ des groupes de formule $-C(=O)-O-(CHZ)_aT$

dans laquelle $a = 0$ à 6

$Z = H$ ou $OH$

$T = H$ ou $OH$

⊖ des groupes de formule $-C(=NH)-O(CHZ)_aT$

dans laquelle $a = 0$ à 3

$Z = H$ ou $OH$

$T = H$ ou $OH$

⊖ le groupe $-C \equiv N$

2) Groupes de type Y. (bivalents)

a) des groupes liés par un carbone tels que

⊖ des groupes de formule 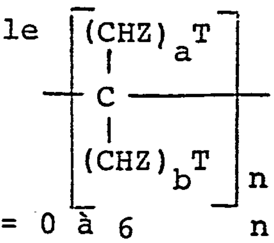

dans laquelle a, b = 0 à 6 n = 1 à 4

Z = H ou OH

T = H ou OH

⊖ le groupe 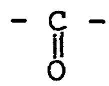

⊖ des groupes de formule 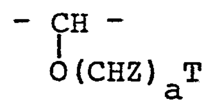

dans laquelle a = O à 6

Z = H ou OH

T = H ou OH

⊖ le groupe 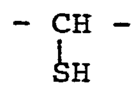

b) des groupes d'autre type tels que

⊖ le groupe 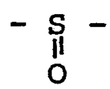

⊖ des groupes de formule

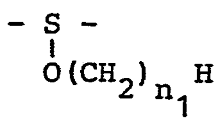 avec $n_1$ = 1 à 6

⊖ le groupe oxy - O -

⊖ des groupes de formule

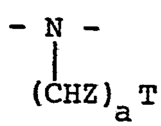

dans laquelle a = 0 à 3

Z, T = H ou OH

⊖ des groupes de formule

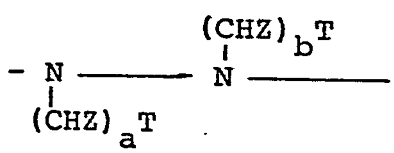

dans laquelle a, b = 0 à 3

Z, T = H ou OH

3) <u>Groupes de type P.</u>

Les groupes P divalents peuvent répondre aux formules générales

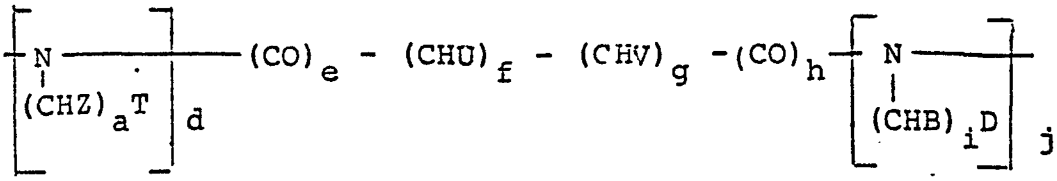

ou 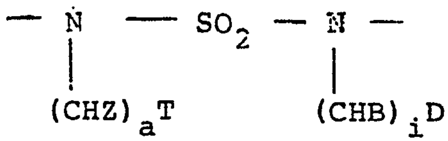

dans lesquelles

d, e, h, j = 0, 1 ou 2

a, f, g, i = 0 à 6

Z, U, B, V = H, OH, Br, $(CH_2)_nCOOH$ avec n = 0 à 3

T, D = H ou OH

D'autres restes peuvent être intercalés dans le pont, tels que les groupes de formules -O- , -S-

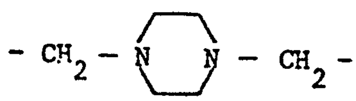

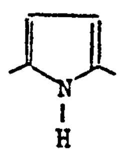 ; 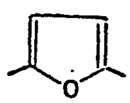 ; 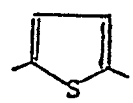

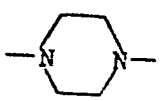 , 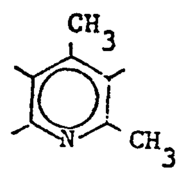

Les groupes P trivalents peuvent être des groupes de formule

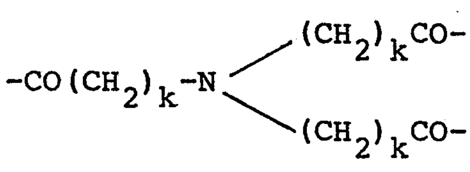

avec k = 0 à 4.

Un groupe préféré de composés selon l'invention sont les composés de formule

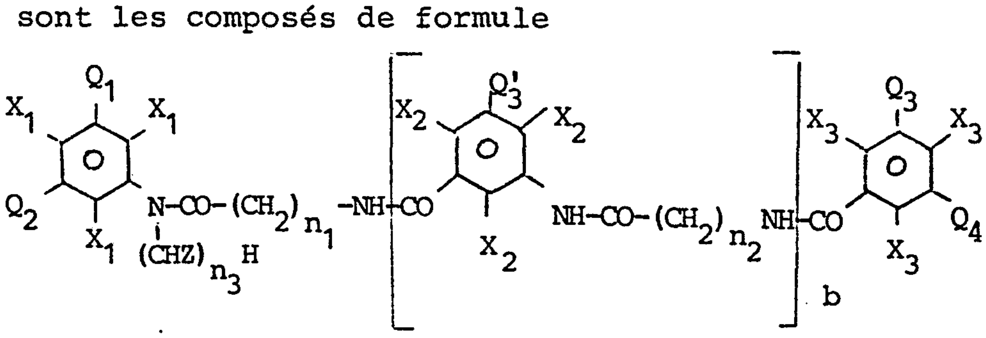 V

dans laquelle

$X_1$, $X_2$ et $X_3$ représentent Br ou I, l'un au moins étant différent des deux autres,

$Q_1$ est un groupe -COOH ou un groupe de formule

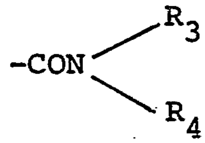, $R_3$ et $R_4$ étant indépendamment l'un de l'autre un atome d'hydrogène, un radical alcoyle inférieur, alcoyle inférieur mono- ou polyhydroxylé ou un radical carboxy alcoyle (inf.),

$Q_2$ représente un atome d'hydrogène, un radical $CH_2OH$, un radical cyano, un radical de formule

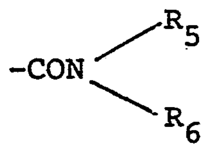, $R_5$ et $R_6$ étant indépendamment l'un de l'autre un atome d'hydrogène, un radical alcoyle inférieur, alcoyle inférieur mono- ou polyhydroxylé ou alcanoyl (inf.) oxyalcoyle (inf.), un groupe amino ou un radical de formule 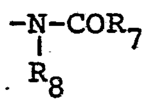, $R_7$ étant un radical alcoyle inférieur, hydroxyalcoyle inférieur ou alcoxy(inf) alcoyle (inf) et $R_8$ un atome d'hydrogène, un radical alcoyle inférieur ou hydroxyalcoyle inférieur,

$Q_3$ et $Q_3'$ représentent indépendamment l'un de l'autre un atome d'hydrogène, un radical cyano, un radical $CH_2OH$, un radical de formule

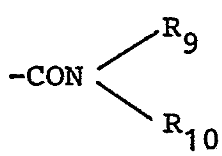, dans laquelle $R_9$ et $R_{10}$ ont les significations données pour $R_5$ et $R_6$, un groupe amino ou un radical de formule 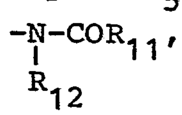, dans laquelle $R_{11}$ a la signification donnée pour $R_7$, et $R_{12}$ a la signification donnée pour $R_8$,

$Q_4$ représente un groupe amino, un radical cyano ou un groupe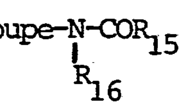

$R_{15}$ étant un radical alcoyle inférieur ou un radical alcoyle inférieur mono-ou polyhydroxylé ou un radical alcoxy (inf.)

 0074307

alcoyle (inf), $R_{16}$ étant un atome d'hydrogène, un radical alcoyle inférieur, un radical hydroxyalcoyle inférieur ou un radical alcanoyle inférieur,

Z est H ou OH

$n_1$ et $n_2$ = 1 à 5

$n_3$ = 0 à 3, et

b = 0 ou 1,

et leurs sels pharmaceutiquement acceptables.

Dans la présente demande, le terme "inférieur" désigne généralement des radicaux ayant de 1 à 6 atomes de carbone.

Pour préparer les composés de formule V, on peut opérer comme décrit dans le brevet US 4 014 986, par condensation d'une amine de formule (VI)

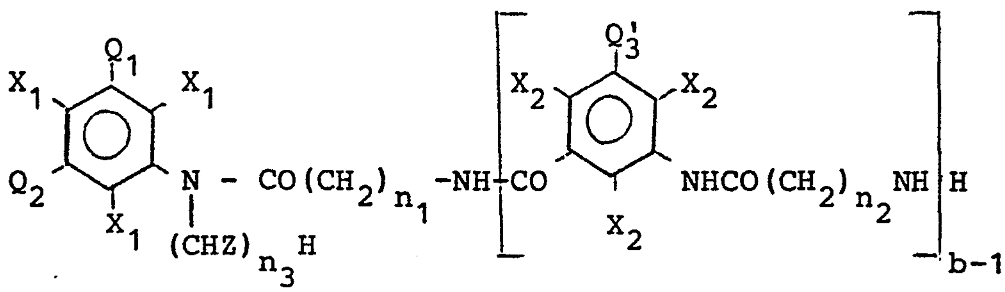

avec un chlorure d'acide de formule

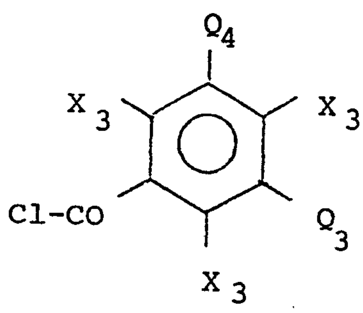

(VII)

notamment dans un solvant polaire à une température de 20 à 60°C en présence d'un accepteur d'acide.

Plus généralement, les composés selon la présente invention peuvent être préparés selon les procédés utilisés pour préparer les analogues polyiodés. A cet effet, on peut mettre en oeuvre notamment des réactions d'halogénation, d'alcoylation, d'acylation (par condensation d'un

chlorure d'acide sur une amine ou un alcool) ou de salification largement décrite pour les analogues polyiodés.

Ainsi, par exemple, les composés de formule générale

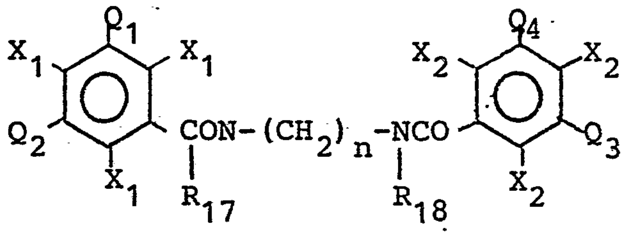

(VIII)

dans laquelle $X_1$ est Br et $X_2$ est I ou $X_1$ est I et $X_2$ est Br,

$Q_1$ et $Q_4$ représentent indépendamment l'un de l'autre un radical amino ou un radical de formule $-\underset{R_{20}}{N}-COR_{19}$ dans laquelle $R_{19}$ représente un radical alcoyle inférieur, alcoyle (inf) mono ou polyhydroxylé, ou alcoxy(inf) alcoyle (inf) et $R_{20}$ un atome d'hydrogène, un radical alcoyle inférieur ou hydroxyalcoyle inférieur,

$Q_2$ et $Q_3$ représentent indépendamment l'un de l'autre un atome d'hydrogène, un radical $CH_2OH$, un radical de formule

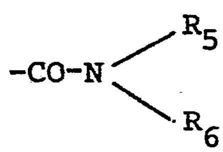

, $R_5$ et $R_6$ étant un atome d'hydrogène, un radical alcoyle inférieur, alcoyle inférieur mono- ou polyhydroxylé ou alcanoyl (inf) oxyalcoyle (inf.) un groupe amino ou un radical de formule $-\underset{R_8}{N}-COR_7$,

$R_7$ étant un radical alcoyle inférieur, alcoyle inférieur mono ou poly hydroxylé ou alcoxy(inf) alcoyle (inf) et $R_8$ un atome d'hydrogène, un radical alcoyle inférieur ou hydroxyalcoyle inférieur, et

$R_{17}$ et $R_{18}$ représentent indépendammant l'un de l'autre un atome d'hydrogène, un radical alcoyle inférieur ou hydroxy alcoyle inférieur et

<u>n</u> est un nombre entier de 1 à 5

peuvent être préparés par condensation d'une amine de formule

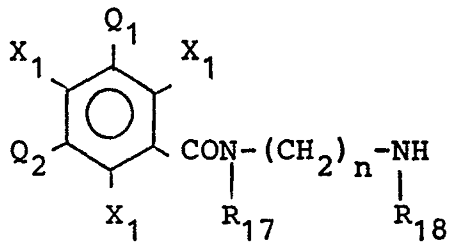

(IX)

sur un chlorure d'acide de formule

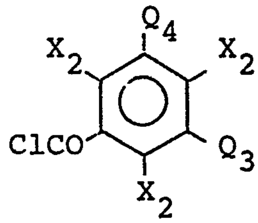

(X)

cette réaction étant suivie éventuellement d'une réaction d'acylation.

Les conditions opératoires peuvent être celles décrites dans le brevet FR 2 313 018.

De même, les composés de formule

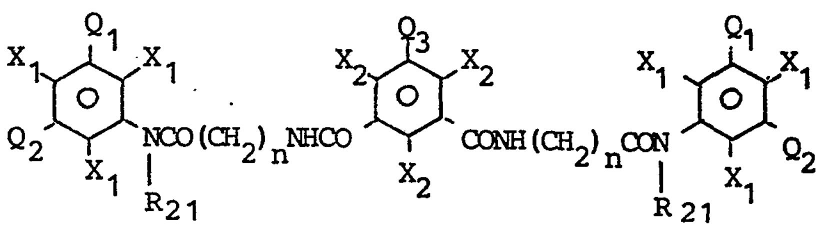

(XI)

dans laquelle $X_1$ est Br et $X_2$ est I ou $X_1$ est I et $X_2$ est Br

$Q_1$ représente un groupe COOH

$Q_2$ représente un atome d'hydrogène, un radical $CH_2OH$, un radical de formule

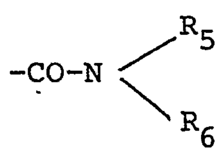

, $R_5$ et $R_6$ étant un atome d'hydrogène, un radical alcoyle inférieur, alcoyle inférieur mono- ou polyhydroxylé ou alcanoyl (inf) oxyalcoyle (inf.) un groupe amino ou un radical de formule 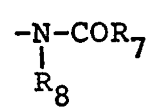 ,

$R_7$ étant un radical alcoyle inférieur, hydroxyalcoyle inférieur ou alcoxy(inf) alcoyle(inf) et $R_8$ un atome d'hydrogène,

un radical alcoyle inférieur ou hydroxyalcoyle inférieur,

$Q_3$ représente un radical de formule $-\underset{R_{12}}{\underset{|}{N}}-COR_{11}$

dans laquelle $R_{11}$ a la signification donnée pour $R_7$ et $R_{12}$ a la signification donnée pour $R_8$,

$R_{21}$ représente un atome d'hydrogène, un radical alcoyle inférieur ou hydroxyalcoyle inférieur, et

n est un nombre entier de 1 à 5

et leurs sels pharmaceutiquement acceptables, peuvent être préparés par condensation d'une amine de formule

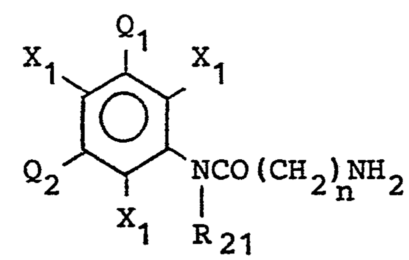

(XII)

sur un dichlorure d'acide de formule

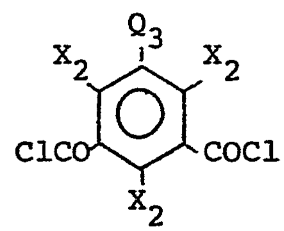

(XIII)

cette réaction étant suivie éventuellement d'une réaction d'acylation et/ou de salification.

Les conditions opératoires peuvent être celles décrites dans le brevet FR 2 347 339.

On donnera ci-après des exemples de préparation des composés selon la présente invention.

Dans ces exemples, les produits ont été caractérisés par leur valeur Rf en chromatographie en couche mince sur plaque de silicagel; les éluants suivants sont utilisés:

- Eluant 1 : Benzène/méthyléthylcétone/acide formique 60/25/20)
- Eluant 2 : n-Butanol/acide acétique/eau (50/11/25).

EXEMPLE 1

Préparation de l'acide tribromo-2,4,6 N-hydroxyéthylcarbamoyl-3 (triiodo-2,4,6 N-méthylcarbamoyl-3 N-méthyl N-acétylamino-5 benzoyl) glycylamino-5 benzoïque (composé 1).

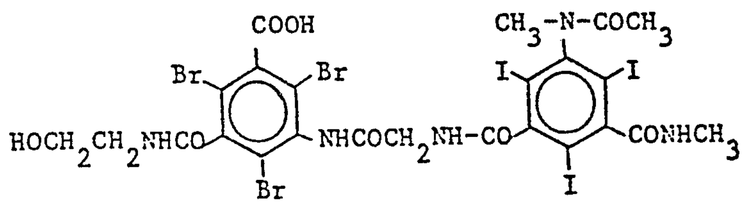

I - Préparation de l'acide tribromo-2,4,6 N-hydroxyéthylcarbamoyl-3 amino-5 benzoïque

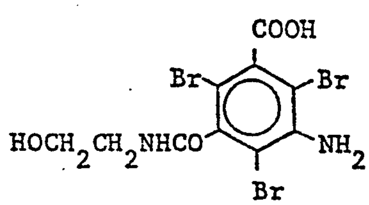

On met en suspension 1 mole d'acide N-hydroxy éthylcarbamoyl-3 amino-5 benzoïque dans 4 litres d'eau et 820 ml d'acide chlorhydrique concentré. On ajoute goutte à goutte 230 ml (9 moles) de brome. On poursuit l'agitation pendant 24 heures à température ambiante. On essore, on lave le précipité dans 2 litres d'eau à 90°C, puis on sèche pendant 24 heures à 110°C.

Rendement : 96,5 %.

Contrôle de pureté :

- CCM dans l'éluant benzène/méthyléthylcétone (MEC)/acide formique 60/25/20
  - . Rf du produit de départ : 0,05
  - . Rf du produit bromé : 0,55
- Pureté d'après le dosage de brome : 100 %.

II - Préparation de l'acide tribromo-2,4,6 N-hydroxyéthyl carbamoyl-3 aminoacétamido-5 benzoïque

a) Préparation de l'acide tribromo-2,4,6 N-phtalimido acétoxyéthylcarbamoyl-3 phtalimidoacétylamino-5 benzoïque.

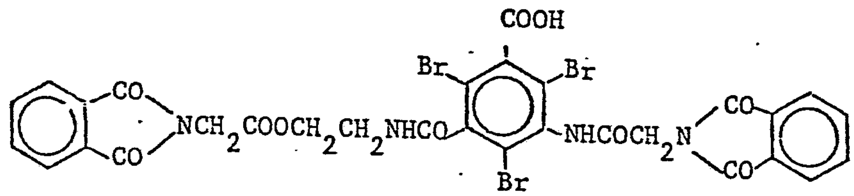

On dissout 322 g (0,7 mole) d'acide tribromo-2,4,6 N-hydroxy éthylcarbamoyl-3 amino-5 benzoïque dans 600 ml de diméthylacétamide. On ajoute par fractions 392 g (1,75 mole) de chlorure d'acide phtalyl glycine. Après 48 heures d'agitation à température ambiante, on verse la solution dans deux litres d'eau à 70°C. Il y a précipitation; on poursuit l'agitation pendant une demi-heure et on essore.

Le produit est utilisé sans séchage et sans purification dans l'étape suivante.

Contrôle :

CCM dans éluant benzène/MEC/acide formique 60/25/20 : Rf 0,7 (produit de départ : Rf 0,55).

b) Préparation de l'acide tribromo-2,4,6 N-hydroxy éthylcarbamoyl-3 aminoacétamido-5 benzoïque.

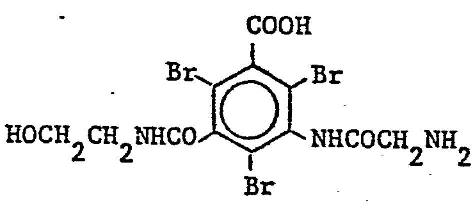

On met en suspension le produit obtenu précédemment dans 2,4 l d'eau et 204 ml d'hydrate d'hydrazine. On chauffe à 90°C pendant 1 heure, on maintient l'agitation pendant 48 heures à température ambiante . Il y a

cristallisation. Ensuite, on essore et on clairce à l'eau. On obtient un produit qui contient 10 à 15 % de phtalhydrazide. Le produit est repris dans 1 litre d'eau et 100 ml d'acide sulfurique concentré; le mélange est porté à 90°C. On sépare l'insoluble par filtration, puis on ajuste le pH à 3-4 par l'ammoniaque. On laisse la cristallisation se poursuivre pendant une nuit à température ambiante.

Après essorage, lavage à l'eau, séchage à l'étuve, on récupère 175 g de produit, soit un rendement de 48,5%.

Contrôle de pureté :

1) CCM dans éluant benzène/MEC/acide formique 60/25/20 : Rf 0,05 (tache jaune orangé par révélation à la ninhydrine.

Il reste environ 1 % de phtalhydrazide à Rf 0,75.

2) Pureté d'après le dosage d'iode : 98 %.

III - Préparation de l'acide tribromo-2,4,6 N-hydroxyéthylcarbamoyl-3 (triiodo-2,4,6 N-méthylcarbamoyl-3 N-méthyl N-acétylamino-5 benzoyl) glycylamino-5 benzoïque

a) Condensation

On met en suspension 75 g (0,145 mole) d'acide tribromo-2,4,6 N-hydroxyéthylcarbamoyl-3 amino acétamido-5 benzoïque dans un mélange de 75 ml de diméthylacétamide et 56 ml (0,4 mole) de triéthylamine. On ajoute ensuite 103 g (0,159 mole) de chlorure d'acide triiodo-2,4,6 N-méthylcarbamoyl-3 N-méthyl N-acétylamino-5 benzoïque. On agite à 45°C pendant 3 heures. On vérifie la fin de réaction par CCM.

La solution obtenue est versée dans 500 ml d'eau et 40 ml d'acide chlorhydrique concentré; il se forme un précipité ; on laisse sous agitation à température ambiante pendant 15 heures. Après essorage, on reprend le produit dans 300 ml d'eau, on le dissout par addition de soude 5N (pH = 7,8), puis on précipite à nouveau par l'acide chlorhydrique 5N. On essore, on lave à l'eau et l'on sèche à l'étuve à 60°C.

On obtient 114,5 g de produit brut, soit un rendement de 70 %.

b) Purification

Elle s'effectue par cristallisation à chaud dans l'éthanol absolu.

114 grammes sont mis en suspension dans 125 ml d'éthanol absolu. On porte au reflux. Il y a dissolution, puis cristallisation. On maintient le chauffage pendant 24 heures. On laisse refroidir. Après essorage, séchage, le produit est dissous dans 250 ml d'eau et de soude. On ajuste à pH = 4-5 par l'acide acétique et l'on fait deux passages au noir 3SA. On filtre, puis on acidifie par l'acide chlorhydrique concentré.

Après essorage, lavage à l'eau et séchage à l'étuve à 60°C, on obtient 54,5 g de produit pur, soit 47,5 % de rendement.

Contrôle de pureté :

1) CCM

- dans éluant benzène/méthyléthylcétone/acide formique 60/25/20 : Rf 0,15.

- dans éluant butanol/acide acétique/eau 50/11/25 Rf 0,3 et 0,38.

2) Pureté d'après le dosage au méthylate : 97 %.

3) Pureté d'après le dosage d'iode : 100 %, d'après le dosage de brome : 100 %.

EXEMPLE 2

Préparation de l'acide triiodo-2,4,6 N-hydroxyéthylcarbamoyl-3 (tribromo-2,4,6 N-méthylcarbamoyl-3 N-méthyl N-acétylamino-5 benzoyl) glycylamino-5 benzoïque (composé 2).

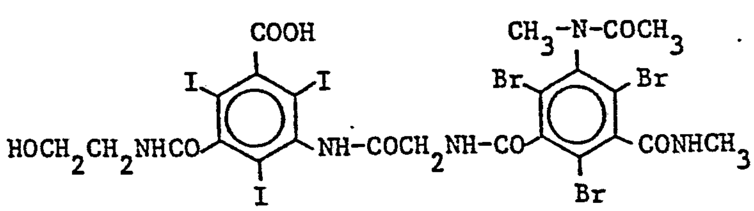

I - Préparation de l'acide tribromo-2,4,6 N-méthylcarbamoyl-3 N-méthyl N-acétylamino-5 benzoïque.

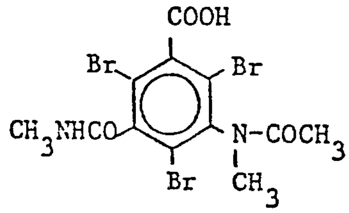

1) Préparation de l'acide tribromo-2,4,6 N-méthylcarbamoyl-3 amino-5 benzoïque.

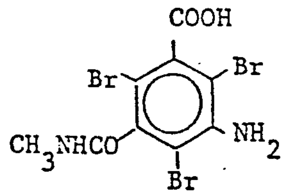

On met en suspension 194 g (1 mole) d'acide N-méthylcarbamoyl-3 amino-5 benzoïque dans 4 litres d'eau et 820 ml d'acide chlorhydrique concentré. On ajoute goutte à goutte 230 ml (9 moles) de brome. On poursuit l'agitation pendant 24 heures à température ambiante. On essore, on lave le précipité dans 2 litres d'eau à 90°C puis on sèche pendant 24 heures à 110°C.

On obtient 410 g d'acide brut, soit un rendement de 95 %.

Contrôle de pureté :

1) CCM dans l'éluant benzène/MEC/acide formique (60/25/20)

.Rf produit de départ : 0,1

.Rf produit bromé : 0,7

2) Pureté d'après le dosage de brome : 98 %.

2) Préparation de l'acide tribromo-2,4,6 N-méthylcarbamoyl-3 N-acétylamino-5 benzoïque.

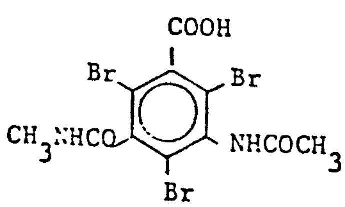

a) Condensation.

On met en suspension 230 g (0,5 mole) d'acide tribromo-2,4,6 N-méthylcarbamoyl-3 amino-5 benzoïque dans 200 ml d'anhydride acétique et 100 ml d'acide acétique. On ajoute goutte à goutte 60 ml d'acide sulfurique concentré (la température ne doit pas dépasser 55-60°C). On agite pendant 1 heure à 55°C à la fin de l'addition d'acide sulfurique. La solution obtenue est versée dans un litre d'eau + glace. Il y a précipitation. On agite à température ambiante pendant 24 heures. On essore, on lave à l'eau. On sèche à l'étuve pendant 16 heures à 80°C.

On obtient 240 g d'acide brut, soit un rendement de 100 %.

b) Purification.

La purification se fait par cristallisation du sel d'ammonium.

On met en suspension 42 g d'acide brut dans 45 ml d'eau. On ajoute de l'ammoniaque 10N jusqu'à dissolution (soit pH = 7-8). On agite pendant 24 heures à température ambiante. Il y a cristallisation. On essore, on clairce par 10 ml d'eau. Le précipité est dissous dans 500 ml d'eau à 90°C. On effectue deux traitements au noir 3SA pendant 2 heures à 80°C. On précipite le produit par l'acide chlorhydrique 1/10. On essore, on lave à l'eau, on sèche à 80°C pendant une nuit.

On obtient 30 g de produit, soit un rendement de 71 %.

Contrôle de pureté :

1) CCM :

- dans l'éluant benzène/MEC/acide formique (60/25/20)

. Rf du produit de départ : 0,7
. Rf du produit acétylé : 0,35

- dans l'éluant butanol/acide acétique/eau (60/11/25)

. Rf du produit de départ : 0,75
. Rf du produit acétylé : 0,3

2) Pureté d'après le dosage de brome : 100 %

3) Pureté d'après le dosage à la soude : 99 %.

3) Préparation de l'acide tribromo-2,4,6 N-méthylcarbamoyl-3 N-méthyl N-acétylamino-5 benzoïque.

a) Méthylation.

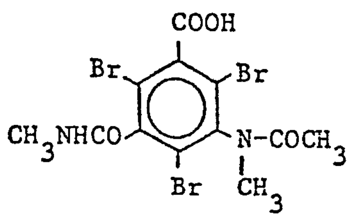

On dissout 189 g (0,4 mole) d'acide tribromo-2,4,6 N-méthylcarbamoyl-3 N-acétylamino-5 benzoïque dans 184 ml de soude 5N (0,92 mole). On ajoute 32,4 ml (0,52 mole) d'iodure de méthyle et on agite pendant 24 heures à température ambiante. La fin de réaction est contrôlée par CCM dans l'éluant : butanol/acide acétique/eau 50/11/25. On verse la solution dans 300 ml d'eau et 75 ml d'acide chlorhydrique concentré. Il y a précipitation. On laisse cristalliser pendant 5 heures, puis on essore. Le précipité est repris dans 500 ml d'eau. On ajoute de la soude 10N jusqu'à dissolution, puis on ramène le pH à 4 par addition d'acide acétique. Pour décolorer la solution on ajoute 1 ml d'une solution de bisulfite de soude. On précipite en milieu acide, on essore, on lave à l'eau et on sèche pendant 24 heures à 80°C.

On obtient 157 g de produit, soit un rendement de 81 % pour la méthylation.

b) Purification.

La purification se fait par recristallisation dans le mélange éthanol-eau.

On met en suspension 100 g d'acide brut dans 500 ml d'eau. On chauffe à 80°C, on ajoute lentement 130 ml d'éthanol à 95 %, soit jusqu'à dissolution complète. On filtre et on laisse recristalliser sous agitation pendant 24 heures. On essore, on clairce par le mélange eau-éthanol et on sèche à l'étuve pendant 24 heures à 80°C.

On obtient 62,8 g de produit que l'on dissout dans 200 ml d'eau et de soude. On ajuste à pH = 4-5 par l'acide acétique et on fait deux passages au noir. On filtre, puis on acidifie par l'acide chlorhydrique concentré. Après essorage, lavage à l'eau, séchage à 80°C pendant 24 heures, on obtient 50 g de produit pur, soit un rendement de 50 %.

Contrôle de pureté :

1) CCM dans l'éluant butanol/acide acétique/eau : 50/11/25

. Rf du produit de départ : 0,3

. Rf du produit méthylé : 0,25 et 0,35.

2) Pureté d'après le dosage au méthylate: 97%

3) Pureté d'après le dosage de brome : 97%.

II - Préparation du chlorure d'acide tribromo-2,4,6 N-méthylcarbamoyl-3 N-méthyl N-acétylamino-5 benzoïque

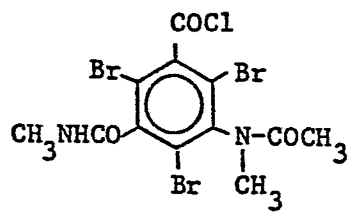

On met en suspension 296 g (0,59 mole) d'acide tribromo-2,4,6 N-méthylcarbamoyl-3 N-méthyl N-acétylamino-5 benzoïque dans 600 ml de chlorure de thionyle. On chauffe à 80°C avec agitation pendant 3 heures. On obtient une solution; on évapore sous vide le chlorure de thionyle en excès. Le résidu pâteux est repris dans 500 ml d'éther isopropylique; on agite à température ambiante pendant 24 heures. Il y a cristallisation. Après essorage, claircage par l'éther isopropylique, on lave le précipité sous agitation pendant 24 heures à température ambiante dans 250 ml d'acétone. On essore, on clairce par 50 ml d'acétone, puis on sèche le produit sous vide.

On obtient 150 g de produit beige clair, soit un rendement de 50,5 %.

Contrôle de pureté :

1) CCM (après réaction avec la monoéthanolamine, en excès dans le diméthylacétamide) dans éluant benzène/méthyléthylacétone/acide formique 60/25/20 :

. Rf du produit de départ 0,57

. Rf du produit condensé avec la monoéthanolamine 0,35

2) Dosage de chlorure d'acide par la propylamine : 105 %.

III - Préparation de l'acide triiodo-2,4,6 N-hydroxyéthylcarbamoyl-3 (tribromo-2,4,6 N-méthylcarbamoyl-3 N-méthyl N-acétylamino-5 benzoyl) glycylamino-5 benzoïque

L'acide triiodo-2,4,6 N-hydroxyéthylcarbamoyl-3 (tribromo-2,4,6 N-méthylcarbamoyl-3 N-méthyl N-acétylamino-5 benzoyl) glycylamino-5 benzoïque est préparé comme dans l'exemple 1-III à partir de l'acide triiodo-2,4,6 N-hydroxyéthylcarbamoyl-3 amino acétamido-5 benzoïque et du chlorure d'acide tribromo-2,4,6 N-méthylcarbamoyl-3 N-méthyl N-acétylamino-5 benzoïque.

Rendement brut : 55 % - Rendement global: 21 %.

Contrôle de pureté :

1) CCM :

. dans éluant benzène/MEC/acide formique 60/25/20 : Rf 0,3.

. dans éluant butanol/acide acétique/eau 50/11/25 : Rf 0,2-0,3.

2) Pureté par dosage au méthylate : 99,6 %.

3) Pureté par dosage d'iode : 98,3 % - par dosage de brome : 99,4 %.

EXEMPLE 3

Préparation de l'acide tribromo-2,4,6 N-hydroxyéthyl carbamoyl-3 (triiodo-2,4,6 N-hydroxyéthyl carbamoyl-3 N-méthyl N-acétylamino-5 benzoyl) glycylamino-5 benzoïque (composé 3).

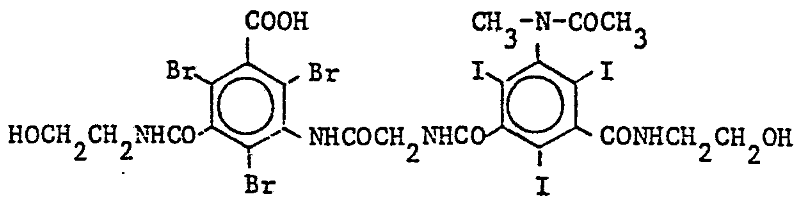

I) Préparation de l'acide triiodo-2,4,6 N-acétoxy éthyl carbamoyl-3 N-méthyl N-acétylamino-5 benzoïque

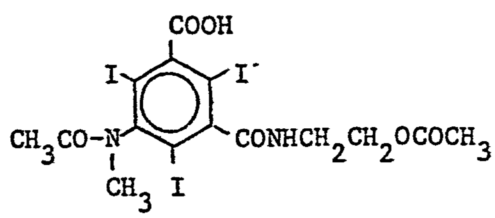

900 g (1,38 mole) d'acide triiodo-2,4,6 N-hydroxy éthyl carbamoyl-3 N-méthyl N-acétylamino-5 benzoïque sont mis en suspension dans 1500 ml de dioxanne. On ajoute 198 ml de chlorure d'acétyle goutte à goutte. Au bout de 4 heures de chauffage à 80°C, on obtient une solution limpide. Après refroidissement, on verse cette solution dans 4,5 l d'éther isopropylique. On obtient des cristaux blancs que l'on essore, lave à l'eau et sèche en étuve à 70°C.

Rendement: approximativement 100 %.

CCM, dans l'éluant 1: Rf 0,45.

II) Préparation du chlorure d'acide triiodo-2,4,6 N-acétoxyéthyl carbamoyl-3 N-méthyl N-acétylamino-5 benzoïque

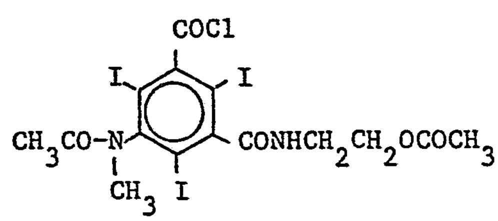

960 g de l'acide précédent sont chauffés au reflux pendant 6 heures dans 2080 ml de chlorure de thionyle. Le chlorure d'acide cristallisé dans le chlorure de

thionyle est essoré après refroidissement et lavé à l'éther isopropylique.

Rendement : 55 %.

Contrôle de pureté :

- Dosage de chlore organique : 101 %
- CCM dans l'éluant 1 : Rf 0,6 après condensation avec la propylamine.

III) Condensation

L'acide tribromo-2,4,6 N-hydroxyéthyl carbamoyl-3 (triiodo-2,4,6 N-acétoxyéthyl carbamoyl-3 N-méthyl N- acétylamino-5 benzoyl) glycyl amino-5 benzoïque de formule

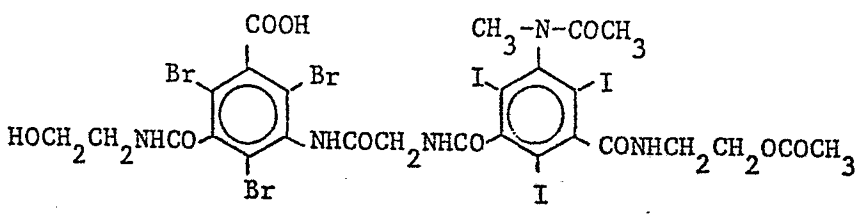

est préparé comme dans l'exemple 1 à partir de l'acide tribromo-2,4,6 N-hydroxyéthyl carbamoyl-3 amino acétamido-5 benzoïque et du chlorure d'acide triiodo-2,4,6 N-acétoxyéthyl carbamoyl-3 N-méthyl N-acétylamino-5 benzoïque.

Rendement de condensation : 70 %.

Contrôle de pureté par CCM dans l'éluant 1 : Rf 0,1.

IV) Saponification

Le produit précédent est dissous dans 320 ml de soude 2N. On chauffe à 50°C pendant 2 heures, puis le produit est précipité à l'acide chlorhydrique, essoré, lavé à l'eau, séché en étuve à 70°C.

Rendement : 65 %.

V) Purification

Elle s'effectue par cristallisation du sel de méthylamine dans l'éthanol. Le produit purifié est essoré, puis lavé au propanol à 80°C pendant 2 heures.

Le sel de métylamine est ensuite dissous dans la soude puis décoloré par passages au noir 3 SA à pH=5

pendant 3 heures à 60°C.

Le produit purifié, essoré, est lavé à l'eau jusqu'à absence de chlorure et de trace de méthylamine.

Contrôle de pureté :

1) CCM :

- Dans l'éluant 1 Rf 0,1.
- Dans l'éluant 2 Rf 0,25 et 0,3.

2) Pureté d'après le dosage d'iode : 97 %.

3) Pureté d'après le dosage de brome : 96 %.

EXEMPLE 4

Préparation de l'acide tribromo-2,4,6 N-hydroxyéthyl carbamoyl-3 (triiodo-2,4,6 N-hydroxyéthylcarbamoyl-3 N-acétylamino-5 benzoyl) glycyl N-méthylamino-5 benzoïque (composé 4)

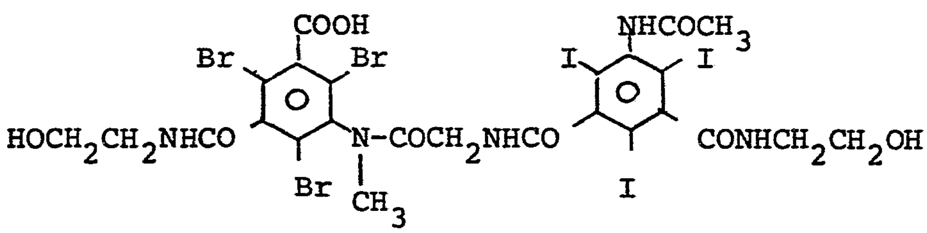

I - Préparation de l'acide tribromo-2,4,6 N-hydroxyéthylcarbamoyl-3 chloro acétamido-5 benzoïque

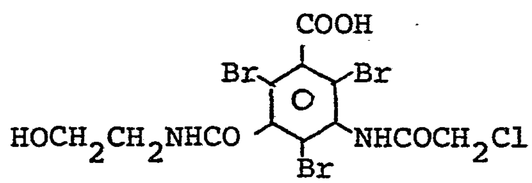

A une suspension d'une mole (461 g) d'acide tribromo-2,4,6 N-hydroxyéthyl carbamoyl-3 amino-5 benzoïque dans 1 l de dioxanne, on ajoute, goutte à goutte, 3 moles de chlorure de chloracétyle.

On chauffe 3 heures à 80°C. Le produit se dissout, puis recristallise. Le produit amidifié et estérifié par le chlorure d' acétyle est essoré et lavé à l'eau. Puis on saponifie la fonction ester par 2 moles de carbonate de sodium dans l'eau à température ambiante. Le produit obtenu est alors précipité par l'acide chlorhydrique, essoré et lavé à l'eau.

Rendement : 78 %.
CCM , éluant 1 : Rf 0,35.
CCM , éluant 2 : Rf 0,5.
% Cl trouvé : 6,55 % Cl théorique : 6,60
% Br trouvé : 44,65 % Br théorique : 44,65

II - Préparation de l'acide tribromo-2,4,6 N-hydroxyéthyl carbamoyl-3 iodo N-méthyl acétamido-5 benzoïque.

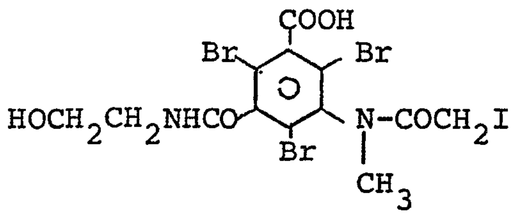

0,75 mole de l'acide précédent est dissoute dans 2,23 moles de soude 5N et 1,5 mole de $CH_3I$. Après 4 heures de chauffage à 40°C, la solution est versée dans 400 ml de HCl industriel dilué au 1/2 par de l'eau glacée, le précipité jaune clair obtenu est essoré et lavé à l'eau.
Rendement : 85 %
Dosage d'acidité : 97,8 %
Dosage d'iode : % I trouvé : 19,35 % I théorique: 19,75
brome : % Br " : 36,55 % Br " : 37,3
CCM , éluant 1 : Rf 0,4

III - Préparation de l'acide tribromo-2,4,6 N-hydroxyéthyl carbamoyl-3 amino N-méthyl acétamido-5 benzoïque.

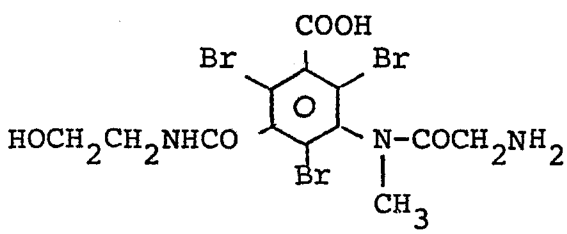

0,517 mole du produit précédent est chauffée 2 heures à 60°C dans 1,5 l de solution d'ammoniaque 10N.

Puis, l'ammoniaque est évaporée sous vide de la trompe à eau. On obtient 350 g du mélange équimolaire de sel d'ammonium du produit attendu et d'iodure

d'ammonium.

CCM , éluant 1 : Rf ≃ 0,05.

Ce produit sera utilisé sans purification.

IV - Condensation

L'acide tribromo-2,4,6 N-hydroxyéthyl carbamoyl-3 (triiodo-2,4,6 N-acétoxyéthyl carbamoyl-3 amino-5 benzoyl) glycyl N-méthyl amino-5 benzoïque de formule

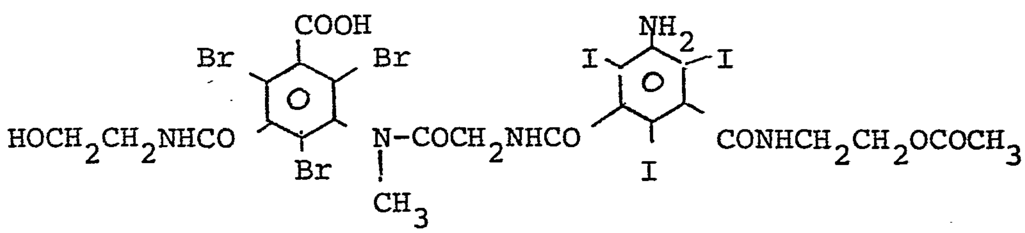

est préparé à partir de l'acide tribromo-2,4,6 N-hydroxyéthyl carbamoyl-3 amino N-méthyl acétamido-5 benzoïque et du chlorure d'acide triiodo-2,4,6 N-acétoxyéthyl carbamoyl-3 amino-5 benzoïque.

Rendement de condensation : 81 %.

CCM , éluant 1 : Rf 0,1

Ce produit est utilisé sans purification.

V - Acétylation

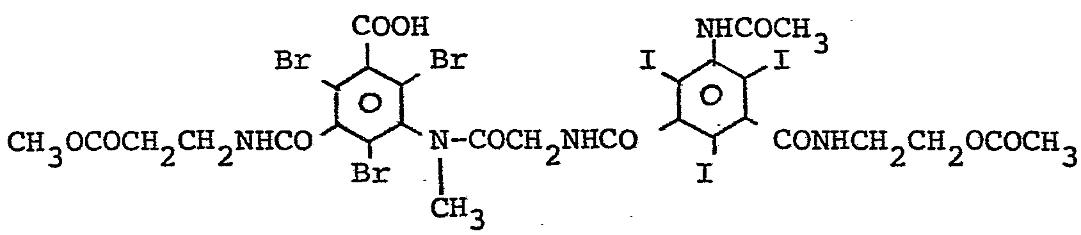

357 g d'acide brut précédent sont dissous dans 330 ml de diméthylacétamide. 1,23 mole de chlorure d'acétyle est ajoutée, goutte à goutte. La solution obtenue est agitée 4 heures à température ambiante, puis versée dans l'eau. Le précipité obtenu est essoré et lavé à l'eau.

Le produit obtenu sera utilisé tel quel, sans purification, ni séchage.

VI - Saponification des fonctions ester

L'acide brut précédent est dissous dans 1,23 mole de soude 2N. La solution alcaline obtenue est agitée à température ambiante pendant 2 heures, puis amenée à pH 5 par de l'acide acétique et on effectue deux passages au noir 3SA à 70°C. La solution filtrée, bien décolorée, est versée sur une solution d'acide chlorhydrique. Le produit attendu précipite; il est séparé par essorage et lavages à l'eau.

Rendement: 53,5 % pour acétylation + saponification.

Purification par sel d'ammonium

Le produit est purifié par deux sels d'ammonium successifs, puis lavé à l'eau, afin d'éliminer les chlorures et les ions $NH_4^+$.

Dosages : acidité : 101,50 %.

| | | |
|---|---|---|
| Brome : | Trouvé : 20,4 % | théorique : 20,7 % |
| Iode : | Trouvé : 32,5 % | théorique : 32,9 % |

CCM , éluant 1 : Rf 0,05

CCM , éluant 2 : 2 isomères Rf 0,17 et 0,27.

EXEMPLE 5

Préparation du composé de formule

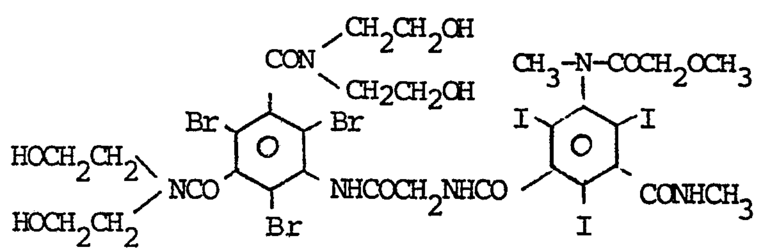

(Composé 25)

I - Condensation de la diéthanol amine sur le dichlorure de l'acide tribromo-2,4,6 aminoisophtalique conduisant

au composé de formule 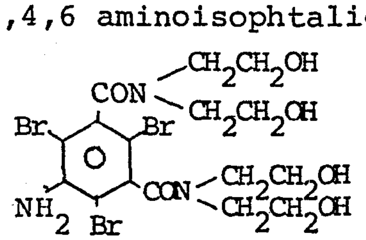

A la solution contenant 1,18 mole de diéthanolamine dans 100 ml d'eau et 1,18 mole de $KHCO_3$, on ajoute goutte à goutte 184 g (0,4 mole) de dichlorure de l'acide tribromo-2,4,6 amino isophtalique en solution dans 185 ml d'acétone.

Du produit blanc précipite peu à peu. Par essorage puis séchage en étuve, on sépare 278 g de produit brut qui migre à Rf 0,1 dans l'éluant 1 et qui contient 9,7% de KCl et 26 % de $KHCO_3$ d'après les dosages de brome, chlore et d'acidité.

Purification par cristallisation dans l'éthanol :

Les 278 g de produit brut sont chauffés à l'ébullition dans 1 litre d'éthanol absolu. 97 g d'insoluble minéral sont éliminés par essorage à chaud. Le produit pur cristallise peu à peu; au bout de deux jours on essore 110 g de produit blanc bien cristallisé.

Contrôle de pureté :

98 % de pureté d'après le dosage de brome.

0 % d'acidité au méthylate.

Pas de $Cl^-$.

CCM dans éluant 1 1 seule tache Rf.0,1.

Rendement : 46,5 % pour condensation + purification.

II - Chloracétylation conduisant au composé de formule :

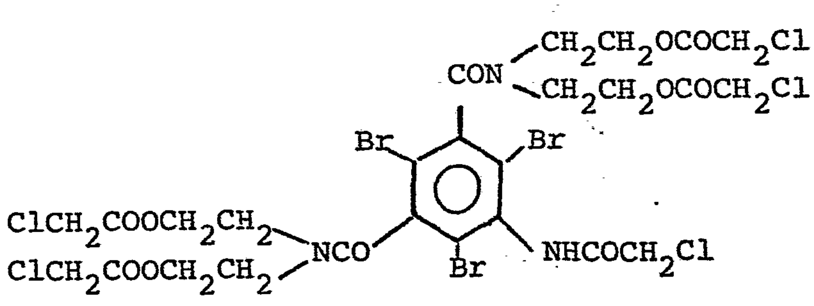

A la suspension de 85 g d'amine précédente, cristallisée dans l'éthanol, dans 100 ml de dioxanne, on ajoute goutte à goutte 80 ml de chlorure de choroacétyle. Au bout d'1/4 d'heure, la dissolution est complète. La solution après une

nuit d'agitation à température ambiante est versée dans 500ml d'eau glacée. On obtient une gomme. La solution surnageante est soutirée et remplacée deux fois par 500ml d'eau glacée. On obtient alors un produit blanc cristallisé.

CCM éluant 1 : Rf 0,6.

III - Saponification des fonctions esters conduisant au composé

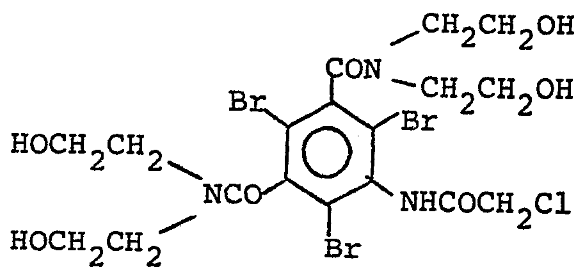

Le produit précédent est dissous dans 1,144 mole de soude 2N et la solution obtenue est agitée 3 h à température ambiante.

On acidifie alors à pH 2 et on extrait l'acide monochloracétique provenant de la saponification par 3 x 250 ml d'acétate d'éthyle jusqu'à pH neutre dans les 2 phases. On obtient 95 g d'un solide blanc cristallisé par évaporation à sec.

CCM éluant 1 : Rf 0,1

Dosage de Br et de Cl organiques : 95 %

IV - Amination conduisant au composé de formule :

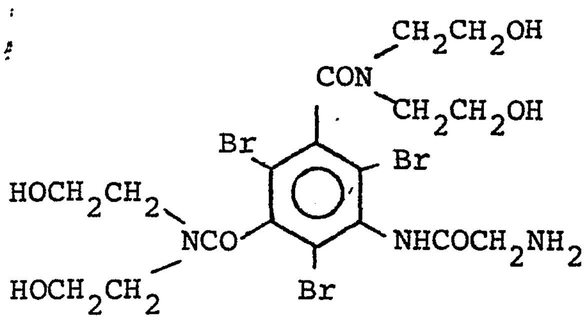

Les 95 g de produit précédent sont dissous dans 600 ml de solution d'ammoniaque 10N. On chauffe à 60° C pendant 3 heures puis on évapore à sec. On obtient un solide presque blanc contenant 1 mole de $NH_4Cl$ pour 1 mole.

CCM éluant 1 : Rf 0

CCM isopropanol/acétate d'éthyle/$NH_4OH$ (35/35/40) : Rf 0,7

CCM éluant 2 : 2 taches Rf 0 et 0,1

Ces taches se révèlent aux U.V. et à la ninhydrine.

V - Condensation

0,154 mole du produit précedent est condensé avec 0,154 mole du chlorure de l'acide triiodo-2,4,6 N-méthyl méthoxy acétamido-3 méthyl carbamoyl-5 benzoïque en présence de 0,23 mole de triéthylamine, dans le DMAC à 45° C. Puis le DMAC est extrait à l'acétate d'éthyle en continu et le produit non ionique est extrait au phénol et la solution aqueuse ainsi obtenue est évaporée. Rendement global :35%.

CCM éluant 1 Rf 0,05 ; éluant 2 Rf 0,3 et 0,45

EXEMPLE 6-

Préparation du composé de formule :

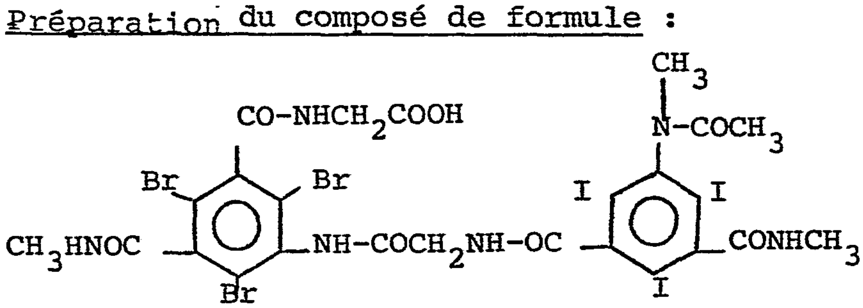

(composé 26)

I - Préparation du composé de formule :

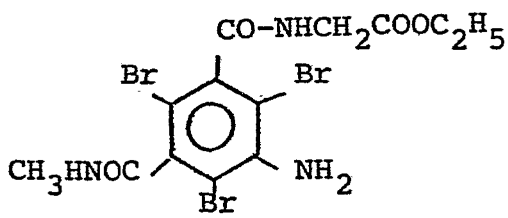

A la suspension de 80 g (0,60 mole) du chlorhydrate d'ester éthylique de la glycine dans 500 ml de DMAC anhydre et 200 ml de triéthylamine on ajoute par portions 176 g (0,39 mole) de chlorure d'acide tribromo amino-5 méthyl-isophtalamique. Après agitation à température ambiante, la solution réactionnelle est versée sur 2 litres d'eau.

Le précipité est essoré, lavé à l'eau, séché.

On obtient 169 g de produit avec un rendement de 84 %.

Pureté (dosage du brome) : 97 %.

II - Préparation du composé de formule :

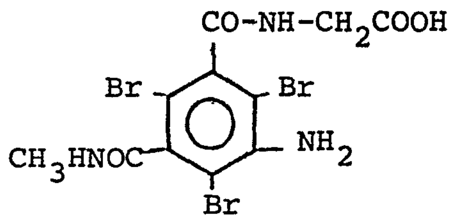

155 g d'ester (0,30 mole) sont agités à température ambiante dans 1 litre de soude normale.

L'acide est obtenu par acidification avec HCl. Les cristaux blancs obtenus sont essorés et lavés à l'eau.

Rendement : 68,5 %

Pureté (-COOH) : 100,6 %.

III - Préparation du composé de formule :

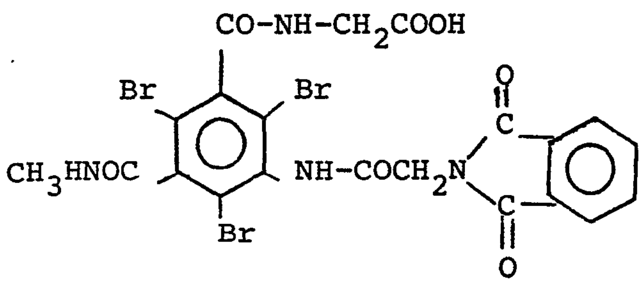

12,2 g du produit précédent (0,025 mole) sont dissous dans 40 ml DMAC anhydre et 6,7 ml de triéthylamine. On ajoute par portions, sous agitation, 9 g (0,04 mole) du chlorure d'acide phtalylglycine. Le milieu réactionnel est versé sur l'eau, le dérivé attendu cristallise.

Rendement : 50 %

Pureté (brome) : 102,8 %.

IV - Préparation du composé de formule :

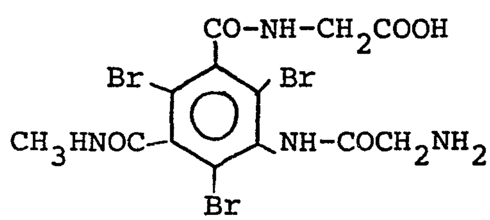

24 g (0,03 mole) du produit précédent sont chauffés dans 50 ml d'eau et 5 ml d'hydrate d'hydrazine pendant 8 heures à 70°.

Le solide obtenu est filtré et séché.

Rendement : 50%

Pureté (brome) : 99%

V - Condensation :

2,2g (0,004 mole) de l'acide amino-acétylé précédemment obtenus sont dissous dans 3ml DMAC + 1,4 ml de triéthylamine + 1ml eau ; on ajoute 2,7 g (0,004 mole) de chlorure d'acide triiodo-2,4,6 méthyl-acétamido-5 isophtalamique par petites portions.Après chauffage pendant 8 heures à 50°C on précipite en milieu acide.Après filtration et séchage on recueille le composé attendu (Rendement = 61%)

CCM (éluant 2) Rf 0,65

EXEMPLE 7 -

Préparation du N-(triiodo-2,4,6 N-hydroxy éthyl carbamoyl-3 N-gluconyl amino-5 benzoyl)N'-(tribromo-2,4,6 N-méthyl acétamido-3 N gluconyl amino-5 benzoyl) diamino-1,2 éthane (composé n°28).

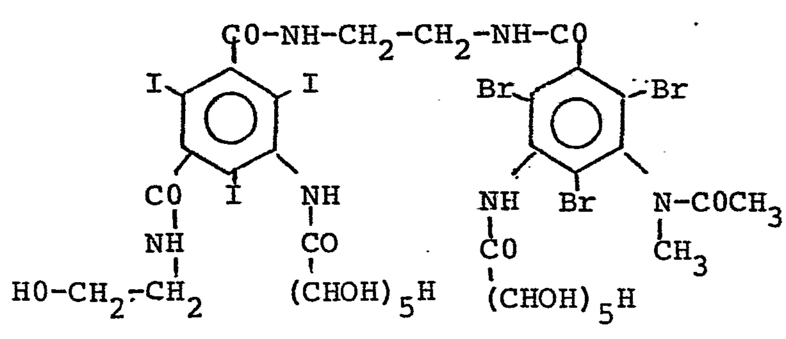

I-Préparation de la (triiodo 2,4,6 N-acétoxy éthyl carbamoyl-3 amino-5) benzoylamino éthylamine :

Le chlorure d'acide triiodo-2,4,6 N-acétoxy éthyl carbamoyl-3 amino-5 benzoïque(216 g,0,325 mole) est mis en suspensic

dans 250 ml d'eau.On ajoute 250 ml d'éthylène diamine en refroidissant de façon à maintenir la température inférieure à 20°C.La dissolution est complète après 2 heures d'agitation.Celle-ci est maintenue 15 heures.Le produit cristallise.Après essorage,lavage au méthanol,on obtient 131 g g de produit soit un rendement de 58,7%.

CCM éluant 1 Rf 0,12

II - Préparation du chlorure d'acide tribromo-2,4,6,N-méthyl acétamido-3 amino-5 benzoïque.

20 g de l'acide correspondant dissous dans 40 ml de $SOCl_2$ sont chauffés 2 heures à 80°.Après évaporation du chlorure de thionyle l'huile est précipitée dans 100 ml d'éther isopropylique.Après agitation 1 heure,essorage, lavage à l'acétate d'éthyle,essorage,séchage on obtient 17 g du composé soit un rendement de 81,5%.

CCM éluant 1 Rf. 0,75

III - Préparation du N-(triiodo-2,4,6,N-acétoxyméthyl, carbamoyl-3 amino-5 benzoyl)N'-(tribromo-2,4,6,N-méthyl acétamido-3 amino-5 benzoyl) diamino-1,2 éthane .

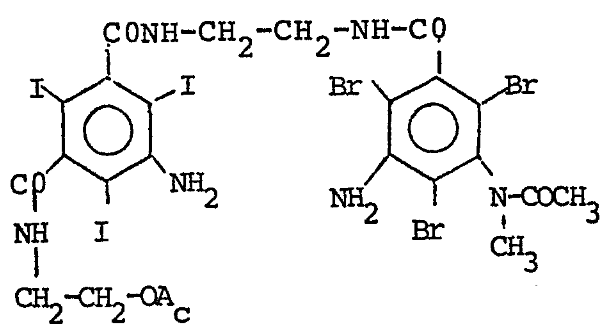

Le composé obtenu en I (100g,0,145 mole) est dissous dans 100 ml de DMAC en présence de triéthylamine (100ml de façon à être à pH basique).Le chlorure d'acide tribromo 2,4,6 N-méthyl acétamido-3 amino-5 benzoïque (80 g,0,168 mole) est ajouté en poudre dans la solution.

Le mélange est chauffé 7 heures à 50° puis agité 8 heures à température ambiante.Après précipitation par $H_2O$ (1 l), essorage,séchage on obtient 170 g du composé soit un rendement de 100 %.

CCM éluant 1 Rf. 0,46

IV - Préparation du N- (triiodo 2,4,6 N-acétoxy éthyl carbamoyl-3 N penta acétoxygluconyl amino-5 benzoyl),N'- (tribromo 2,4,6 N-méthyl acétamido-3, N-penta acétoxygluconyl amino-5 benzoyl) diamino-1,2 éthane

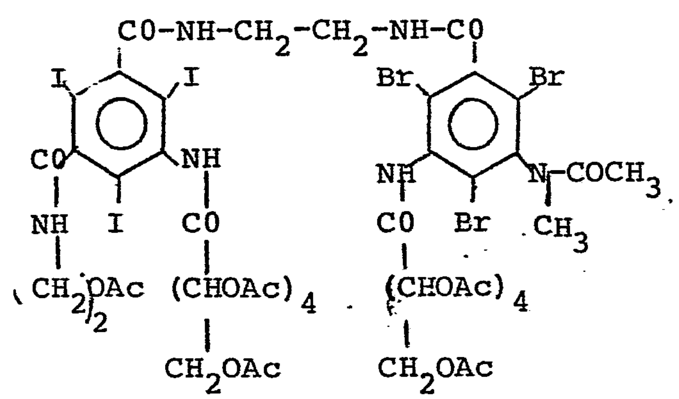

On dissout 100 g (0,089 mole) du composé obtenu ci-dessus dans 180 ml de DMAC.On ajoute 0,445 mole de chlorure d'acide gluconique pentaacétylé en poudre qui se dissout au fur et à mesure.

Après agitation 20 heures à température ambiante le mélange est précipité par 1,3 l d'$H_2O$ glacée. On maintient l'agitation une nuit. Après essorage, le produit gommeux est repris par 300 ml de $CH_2Cl_2$. La phase organique lavée au bicarbonate (5%) puis par $H_2O$ est évaporée. On obtient 126 g de produit beige soit un rendement de 75%.

V - Déprotection -

La déprotection des groupements acétyles s'effectue par transesterification sur 100 g (0,053 mole ) du composé obtenu dans 250 ml de méthanol en présence de 6,2 ml (0,1 mole) d'éthanolamine servant de catalyseur. Après chauffage à 35° (48h) puis agitation à température ambiante (48 heures) le mélange réactionnel est passé sur résine $H^+$. Après concentration du filtrat une précipitation par $H_2O$ permet d'obtenir 26,6 g de produit encore impur. Celui-ci est dissous dans l'eau chaude. Après élimination de l'insoluble, le filtrat concentré permet d'obtenir 4 g du composé attendu soit en rendement de 5,3%.

| | | | | |
|---|---|---|---|---|
| CCM | Eluant 2 Rf. | produit de départ Rf. | 0,70 | 0,86 |
| | | produit obtenu | 0,35 | 0,40 |
| | Eluant 1 Rf. | produit de départ | 0,72 | |
| | | produit obtenu | 0,05 | |

On a rassemblé dans les tableaux I et II ci-après les caractéristiques des composés des exemples 1 à 7 et celles d'autres composés de formule générale V, ainsi que celles de composés de formule III préparés par des procédés analogues.

0074307

TABLEAU I

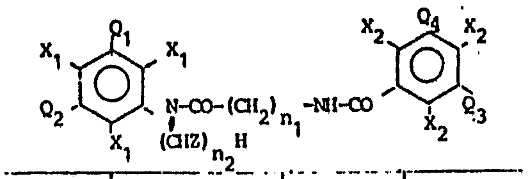 (Va)

| Composé | $X_1$ | $X_2$ | $Q_1$ | $Q_2$ | $Q_3$ | $Q_4$ | $n_1$ | Z | $n_2$ | Rdt % conden-sation | Rdt % saponifi-cation | Rdt % global | Rf Eluant 1 | Rf Eluant 2 |
|---|---|---|---|---|---|---|---|---|---|---|---|---|---|---|
| 1 | Br | I | $-COOH$ | $-CONHCH_2CH_2OH$ | $-CONHCH_3$ | 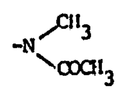 | 1 | - | 0 | 70 | | 33 | 0,15 | 0,3 et 0,38 |
| 2 | I | Br | " | " | " | " | " | - | " | 55 | | 21 | 0,3 | 0,2 et 0,3 |
| 3 | I | | " | " | $-CONHCH_2CH_2OH$ | " | " | - | " | 70 | 65 | | 0,1 | 0,25 et 0,3 |
| 4 | Br | I | " | " | $-CONHCH_2CH_2OH$ | $-NHCOCH_3$ | " | H | 1 | 81 | Ac+Sap. 53,5 | | 0,05 | 0,17 et 0,27 |
| 5 | I | Br | " | $-CONHCH_3$ | H | 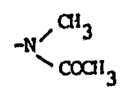 | " | - | 0 | 76 | | 16 | 0,45 | 0,3 et 0,4 |
| 6 | Br | I | " | " | " | " | " | - | " | 88 | | 60 | 0,50 et 0,55 | 0,35 et 0,45 |
| 7 | Br | I | " | H | $-CONHCH_3$ | " | " | - | " | 66 | | 50 | 0,45 | 0,4 et 0,5 |
| 8 | I | Br | " | " | " | " | " | - | " | 83,7 | | 48 | 0,55 | |
| 9 | Br | I | " | $-CONHCH_2CH_2OH$ | " | 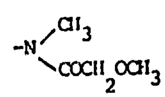 | " | - | " | 87 | | 23,5 | 0,05 | 0,25 |
| 10 | I | Br | " | " | " | " | " | - | " | 47 | | 20 | 0,1 | 0,25 |
| 11 | I | Br | " | " | $-CONHCH_2CH_2OH$ | 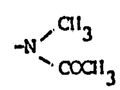 | " | - | " | 90 | 51 | 12,5 | 0,1 | 0,45 et 0,55 |

0074307

TABLEAU I (Suite)

| Composé | $X_1$ | $X_2$ | $Q_1$ | $Q_2$ | $Q_3$ | $Q_4$ | $n_1$ | Z | $n_2$ | Rdt % condensation | Rdt % saponification | Rdt % global | Rf Eluant 1 | Rf Eluant 2 |
|---|---|---|---|---|---|---|---|---|---|---|---|---|---|---|
| 12 | I | Br | $-COOH$ | H | $-CONHCH_3$ | 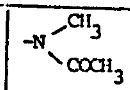 | 2 | - | 0 | 93 | | 56 | 0,4 | 0,45 et 0,6 |
| 13 | I | Br | " | $-CONHCH_2CH_2OH$ | " | " | 3 | - | " | 72 | | 45 | 0,5 | 0,2 et 0,25 |
| 14 | I | Br | " | $-CONHCH_3$ | " | " | 3 | - | " | 84 | | 41 | 0,13 | 0,2 et 0,3 |
| 15 | I | Br | " | $-CONHCH_2CH_2OH$ | $-CONHCH_2CH_2OH$ | $-NHCOCH_3$ | 1 | H | 1 | 85 | Ac+Sap. 61 | 20 | 0,05 | 0,2 et 0,27 |
| 16 | Br | I | " | $-CONHCH_3$ | $-NHCOCH_3$ | 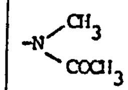 | " | - | 0 | 77,5 | Acétylation 52 | 25 | 0,20 et 0,25 | 0,35 et 0,40 |
| 17 | Br | I | " | 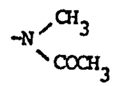 | $-CONHCH_3$ | $-NHCOCH_3$ | " | - | " | | | | 0,25 | |
| 18 | I | Br | " | " | " | " | " | - | " | | | | 0,15 | |
| 19 | I | Br | " | $-NHCOCH_3$ | 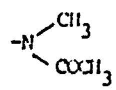 | " | " | - | " | 82 | Actéyl.55,5 | 20 | 0,10 et 0,15 | 0,20 et 0,30 |

TABLEAU I (Suite 2)

| Composé | $X_1$ | $X_2$ | $Q_1$ | $Q_2$ | $Q_3$ | $Q_4$ | $n_1$ | Z | $n_2$ | Rdt % condensation | Rdt % saponification | Rdt % global | Rf. Eluant 1 | Rf Eluant 2 |
|---|---|---|---|---|---|---|---|---|---|---|---|---|---|---|
| 20 | I | Br | $-COOH$ | 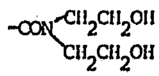 | $-CONHCH_3$ | 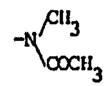 | 1 | - | 0 | 61,5 | | 13,5 | 0,1 | 0,15 et 0;23 |
| 21 | I | Br | " | $-CONHCH_2CH_2OH$ | $-CONHCH_2CH_2OH$ | $-NHCOCH_2OCH_3$ | " | - | " | | | | 0,03 | 0,1 et 0,17 |
| 22 | Br | I | " | " | " | " | " | - | " | | | | 0,05 | 0,1 et 0,17 |
| 23 | I | Br | " | " | " | 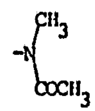 | " | H | 1 | | | | 0,05 | 0,1 et 0,2 |
| 24 | Br | I | " | " | " | " | " | H | 1 | | | | 0,05 | 0,1 et 0,2 |
| 25 | Br | I | 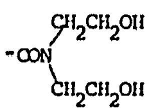 | 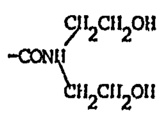 | $-CONHCH_3$ | 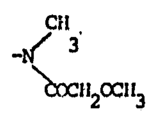 | " | - | 0 | | | 35 | 0,05 | 0.3 et 0,45 |
| 26 | Br | I | $-CONHCH_2\ COOH$ | $-CONHCH_3$ | $-CONHCH_3$ | 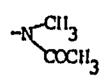 | " | - | " | 61 | | | | 0,65 |
| 27 | I | Br | $-COOH$ | $-CH_2OH$ | $-CONHCH_3$ | 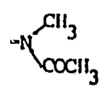 | " | - | " | | | | 0,25 et 0,30 | 0,3 et 0,4 |
| 28 | Br | I | $-COOH$ | $-CONCH_2CH_2OH$ | $-CONHCH_3$ | $-CN$ | 1 | - | " | 70 | | | 0,15 | 0,4 |

TABLEAU II

COMPOSES DE FORMULE III

| Composé | Formules chimiques | Rf Eluant 1 | Rf Eluant 2 | |
|---|---|---|---|---|
| 29 | 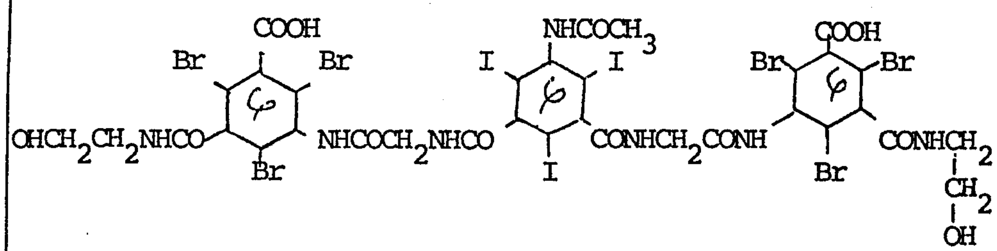 | 0,65 | 0,050 | |
| 30 | 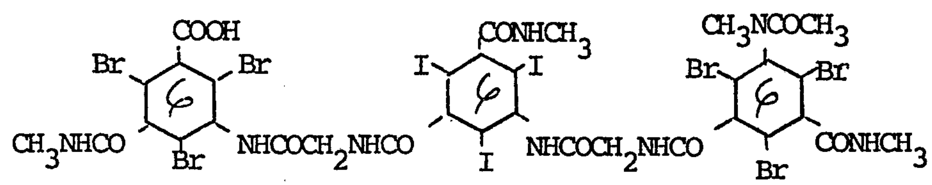 | 0,050 | 0,30 et 0,35 | |
| 31 | 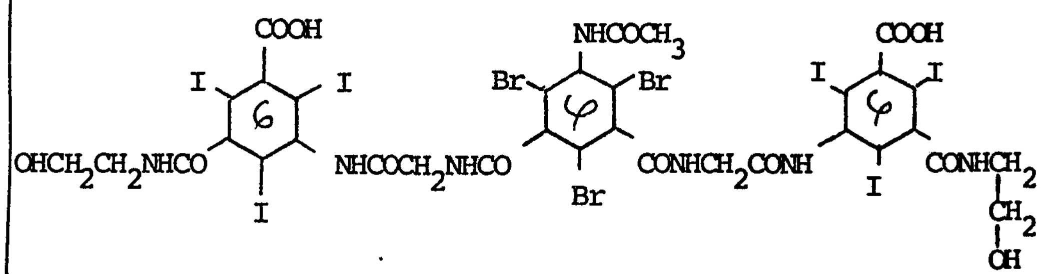 | | 0,10 | |

On donnera ci-après des résultats d'essais comparatifs effectués avec les composés selon la présente invention.

1 - Opacité

L'opacité a été mesurée indirectement à l'aide de l'impression produite sur un film photographique par un faisceau de rayons X traversant une cuve contenant une solution aqueuse des composés. La transparence du film après développement a été mesurée à l'aide d'un densitomètre optique. Dans chaque cas, la mesure a été effectuée sur le même film et avec la même source de rayons X (70 kV) par comparaison avec un produit opacifiant servant de référence.

Comme produit de référence, on a utilisé l'Hexabrix qui est une solution de sels de sodium et de méthylglucamine de l'acide ioxaglique à 32 % en iode.

Les composés selon l'invention ont été testés sous forme de solutions aqueuses de leurs sels de méthylglucamine contenant le même nombre de moles de composés que l'Hexabrix.

Les résultats sont donnés dans le tableau III ci-après .

TABLEAU III

| Composés | Opacité relative/Hexabrix |
|---|---|
| 1 | 0,91 |
| 2 | 0,92 |
| 5 | 0,92 |

On constate que l'opacité des composés selon l'invention est très proche de celle de l'Hexabrix, un composé uniquement iodé de structure semblable à celle des composés testés. Pour obtenir avec les produits selon l'invention la même opacité qu'avec les produits uniquement iodés, il faut seulement 1,09 fois plus de moles de composé.

2 - Activation du complément

Les travaux d'Elliott C. Lasser (Investig. Radiol., 1974, 9, 4 6A) ont montré que les produits de contraste activent le complément, qui est un ensemble de protéines sériques dont l'activation peut conduire à des réactions anaphylactiques graves.

La mesure de cette activation est effectuée par la détermination de la concentration hémolytique 50 désignée CH 50. On estime que les produits moins activateurs du complément, c'est-à-dire ayant une CH 50 plus élevée , provoquent moins d'effets secondaires de type anaphylactiques et présentent donc une meilleure tolérance.

Les résultats sont donnés dans le tableau IV.

TABLEAU IV

| Composé testé | CH 50 moles/ ml |
|---|---|
| 1 | 20,2 |
| 2 | 20,8 |
| Analogue complètement iodé des composés 1 et 2 | 17,9 |
| 5 | 7,4 |
| Analogue complètement iodé du composé 5 | 3,8 |
| 7 | 10,0 |
| 8 | 11,5 |
| 12 | 11,0 |
| 13 | 15,3 |

3 - Toxicité aigüe intracisternale -

La toxicité aigüe intracisternale a été mesurée chez le rat selon la méthode de E.Melartin,P.Tuohimaa,R. Dabb.Investigative Radiology,1970,vol.5,n°1,13-21

Les résultats sont donnés dans le tableau V.

TABLEAU V

| Composé | Toxicité aigüe IC rat |
|---|---|
| 2 | 170 |
| Analogue complètement iodé du composé 2 | 75 |
| 5 | 10,5 |
| Analogue complètement iodé du composé 5 | 3,7 |

Les composés mixtes (bromés et iodés) selon la présente invention peuvent donc être utilisés comme produits spécifiants à des fins radiologiques.

La forme pharmaceutique préférée est constituée par des solutions aqueuses d'un ou plusieurs composés mixtes tels que définis ci-dessus.

Les solutions aqueuses contiennent généralement de 5 à 100 g de composé mixte et la quantité injectable de telles solutions peut varier généralement de 5 à 1000 ml.

0074307

## REVENDICATIONS

1. Composés à structure iodobenzénique, utilisables comme produits opacifiants pour la radiographie, présentant une tolérance plus grande que celle des composés analogues polyiodés et une opacité du même ordre de grandeur que ces composés polyiodés, caractérisés en ce qu'une partie des atomes d'iode nucléaires présents dans les composés analogues polyiodés est remplacée par des atomes de brome.

2. Composés selon la revendication 1, caractérisés en ce que le nombre des atomes de brome nucléaires représente de 1/2 à 2/1 du nombre des atomes d'iode nucléaires.

3. Composés selon la revendication 2, caractérisés en ce qu'ils présentent deux noyaux benzéniques dont l'un est triiodé et dont l'autre est tribromé.

4. Composés selon la revendication 3, caractérisés en ce qu'ils répondent à la formule

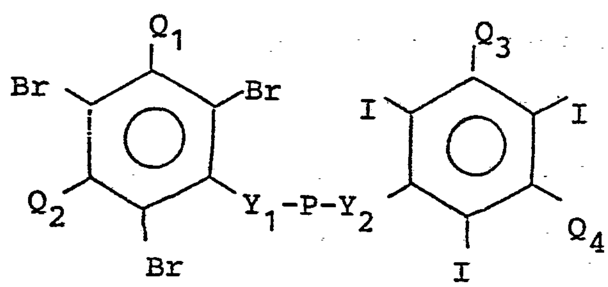

$(II_a)$

dans laquelle

$Q_1$, $Q_2$, $Q_3$, $Q_4$, $Y_1$, $Y_2$ et P sont des groupes pharmacologiquement acceptables.

5. Composés selon la revendication 1, caractérisés en ce qu'ils répondent à la formule

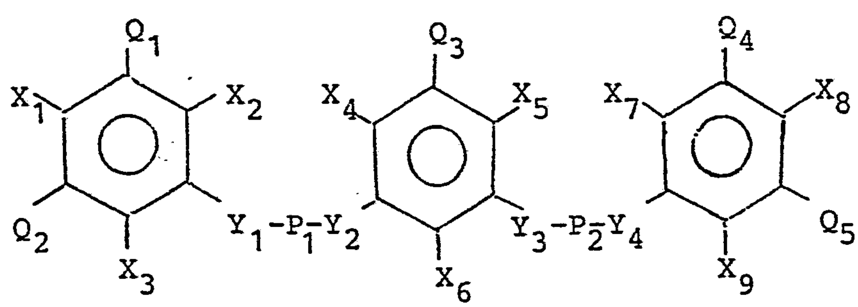

(III)

dans laquelle

$X_1$, $X_2$, $X_3$, $X_4$, $X_5$, $X_6$, $X_7$, $X_8$ sont choisis parmi I et Br, I ou Br étant présents simultanément, et

$Q_1$, $Q_2$, $Q_3$, $Q_4$, $Q_5$, $Y_1$, $Y_2$, $Y_3$, $Y_4$, $P_1$ et $P_2$ sont des groupes pharmaceutiquement acceptables.

6. Composés selon la revendication 1, caractérisés en ce qu'ils répondent à la formule

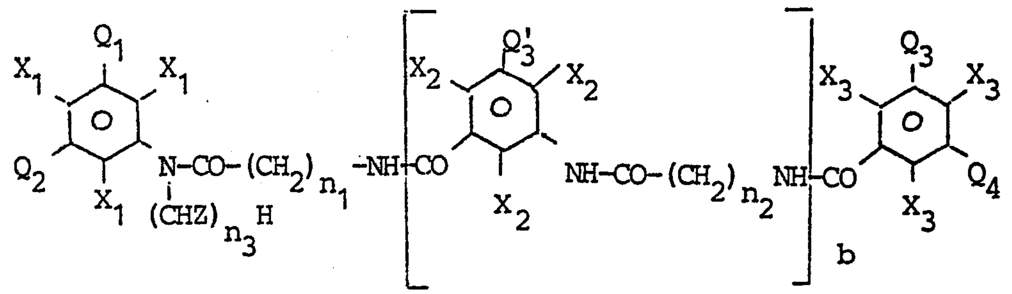

V

dans laquelle

$X_1$, $X_2$ et $X_3$ représentent Br ou I, l'un au moins étant différent des deux autres,

$Q_1$ est un groupe -COOH ou un groupe de formule

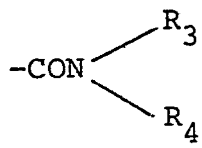 , $R_3$ et $R_4$ étant indépendamment l'un de l'autre un atome d'hydrogène, un radical alcoyle inférieur, alcoyle inférieur mono- ou polyhydroxylé ou un radical carboxy alcoyle (inf.),

$Q_2$ représente un atome d'hydrogène, un radical $CH_2OH$, un radical cyano, un radical de formule

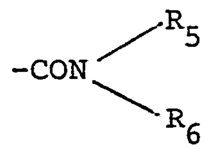 , $R_5$ et $R_6$ étant indépendamment l'un de l'autre un atome d'hydrogène, un radical alcoyle inférieur, alcoyle inférieur mono- ou polyhydroxylé ou alcanoyl (inf.) oxyalcoyle (inf.), un groupe amino ou un radical de formule $-N(R_8)-COR_7$ , $R_7$ étant un radical alcoyle inférieur, hydroxyalcoyle inférieur ou alcoxy(inf) alcoyle (inf) et $R_8$ un atome d'hydrogène, un radical alcoyle inférieur ou hydroxyalcoyle inférieur,

$Q_3$ et $Q_3'$ représentent indépendamment l'un de l'autre un atome d'hydrogène, un radical cyano, un radical $CH_2OH$, un radical de formule

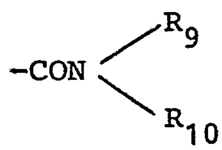 , dans laquelle $R_9$ et $R_{10}$ ont les significations données pour $R_5$ et $R_6$, un groupe amino ou un radical de formule $-N(R_{12})-COR_{11}$, dans laquelle $R_{11}$ a la signification donnée pour $R_7$, et $R_{12}$ a la signification donnée pour $R_8$,

$Q_4$ représente un groupe amino, un radical cyano ou un groupe $-N(R_{16})-COR_{15}$

$R_{15}$ étant un radical alcoyle inférieur ou un radical alcoyle inférieur mono-ou polyhydroxylé ou un radical alcoxy (inf.)

alcoyle (inf), $R_{16}$ étant un atome d'hydrogène, un radical alcoyle inférieur, un radical hydroxyalcoyle inférieur ou un radical alcanoyle inférieur,

Z est H ou OH

$n_1$ et $n_2$ = 1 à 5

$n_3$ = 0 à 3, et

b = 0 ou 1,

et leurs sels pharmaceutiquement acceptables.

7 -Composés selon la revendication 1, caractérisé en ce qu'ils répondent à la formule

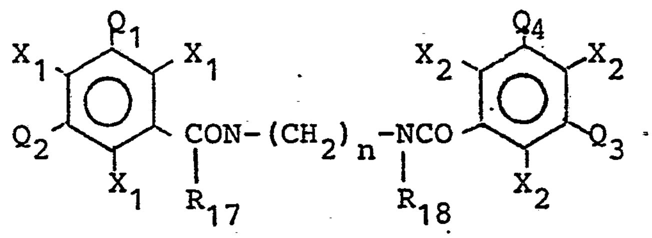

(VIII)

dans laquelle $X_1$ est Br et $X_2$ est I ou $X_1$ est I et $X_2$ est Br

$Q_1$ et $Q_4$ représentent indépendamment l'un de l'autre un radical amino ou un radical de formule 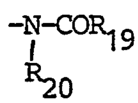 dans laquelle $R_{19}$ représente un radical alcoyle inférieur, alcoyle (inf) mono ou polyhydroxylé, ou alcoxy(inf) alcoyle (inf) et $R_{20}$ un atome d'hydrogène, un radical alcoyle inférieur ou hydroxyalcoyle inférieur,

$Q_2$ et $Q_3$ représentent indépendamment l'un de l'autre un atome d'hydrogène, un radical $CH_2OH$, un radical de formule

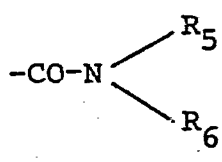 , $R_5$ et $R_6$ étant un atome d'hydrogène, un radical alcoyle inférieur, alcoyle inférieur mono- ou polyhydroxylé ou alcanoyl (inf) oxyalcoyle (inf.) un groupe amino ou un radical de formule 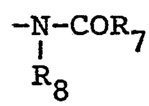,

$R_7$ étant un radical alcoyle inférieur, alcoyle inférieur mono ou poly hydroxylé ou alcoxy(inf) alcoyle (inf) et $R_8$ un atome d'hydrogène, un radical alcoyle inférieur ou hydroxy-alcoyle inférieur, et

$R_{17}$ et $R_{18}$ représentent indépendammant l'un de l'autre un atome d'hydrogène, un radical alcoyle inférieur ou hydroxy alcoyle inférieur et

n est un nombre entier de 1 à 5

8 - Composés selon la revendication 1, caractérisé en ce qu'ils répondent à la formule

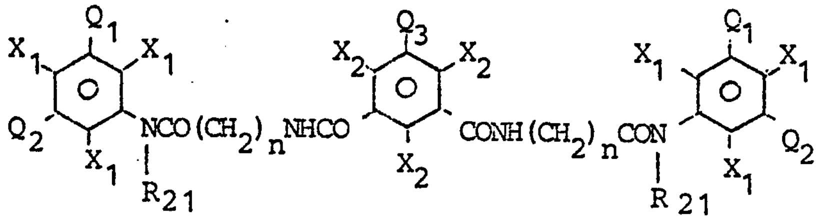

(XI)

dans laquelle $X_1$ est Br et $X_2$ est I ou $X_1$ est I et $X_2$ est Br

$Q_1$ représente un groupe COOH

$Q_2$ représente un atome d'hydrogène, un radical $CH_2OH$, un radical de formule

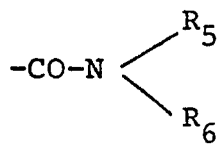

, $R_5$ et $R_6$ étant un atome d'hydrogène, un radical alcoyle inférieur, alcoyle inférieur mono- ou polyhydroxylé ou alcanoyl (inf) oxyalcoyle (inf.) un groupe amino ou un radical de formule $-\underset{R_8}{\underset{|}{N}}-COR_7$ ,

$R_7$ étant un radical alcoyle inférieur, hydroxyalcoyle inférieur ou alcoxy(inf) alcoyle(inf) et $R_8$ un atome d'hydrogène, un radical alcoyle inférieur ou hydroxyalcoyle inférieur,

$Q_3$ représente un radical de formule $-\underset{R_{12}}{\underset{|}{N}}-COR_{11}$

dans laquelle $R_{11}$ a la signification donnée pour $R_7$ et $R_{12}$ a la signification donnée pour $R_8$,

$R_{21}$ représente un atome d'hydrogène, un radical alcoyle inférieur ou hydroxyalcoyle inférieur, et

<u>n</u> est un nombre entier de 1 à 5

et leurs sels pharmaceutiquement acceptables,

9-Produit opacifiant pour la radiographie, caractérisé en ce qu'il contient au moins un composé tel que défini à l'une quelconque des revendications 1 à 8.

10-Produit opacifiant selon la revendication 9, caractérisé en ce qu'il est constitué d'une solution aqueuse du composé ou des composés.

Office européen des brevets

**RAPPORT DE RECHERCHE EUROPEENNE**

0074307
Numéro de la demande

EP 82 40 1577

| DOCUMENTS CONSIDERES COMME PERTINENTS | | | |
|---|---|---|---|
| Catégorie | Citation du document avec indication, en cas de besoin, des parties pertinentes | Revendication concernée | CLASSEMENT DE LA DEMANDE (Int. Cl. ³) |
| | --- | | |
| A | FR-A-2 272 640 (LABS ANDRE GUERBET) | 1-10 | C 07 C 103/84<br>C 07 C 103/82<br>A 61 K 49/04 //<br>C 07 C 103/78<br>C 07 C 103/76<br>C 07 C 103/46<br>C 07 C 121/52<br>C 07 D 209/48 |
| | --- | | |
| A | FR-A-2 347 339 (GUERBET S.A.) | 1-10 | |
| | --- | | |
| A | FR-A-2 313 018 (LABS ANDRE GUERBET) | 1-10 | |
| | ----- | | |
| | | | DOMAINES TECHNIQUES RECHERCHES (Int. Cl. ³) |
| | | | A 61 K 49/00<br>C 07 C 103/00 |

Le présent rapport de recherche a été établi pour toutes les revendications

| Lieu de la recherche | Date d'achèvement de la recherche | Examinateur |
|---|---|---|
| LA HAYE | 25-11-1982 | PAUWELS G.R.A. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503. 03.82